(19) Europäisches Patentamt
European Patent Office
Office européen des brevets

(11) Numéro de publication: **0 379 441 A1**

# (12) DEMANDE DE BREVET EUROPEEN

(21) Numéro de dépôt: **90400153.4**

(22) Date de dépôt: **19.01.90**

(51) Int. Cl.5: **C07D 405/06, C07D 405/12, C07D 311/58, A61K 31/395**

(30) Priorité: **20.01.89 FR 8900657**

(43) Date de publication de la demande: **25.07.90 Bulletin 90/30**

(84) Etats contractants désignés: **AT BE CH DE DK ES FR GB GR IT LI LU NL SE**

(71) Demandeur: **RHONE-POULENC SANTE**
**20, avenue Raymond Aron**
**F-92165 Antony Cédex(FR)**

(72) Inventeur: **Barreau, Michel**
**24bis Avenue du Clos de Sénart**
**F-91230 Montgeron(FR)**
Inventeur: **Hardy, Jean-Claude**
**1 Passage des Griottes**
**F-95800 Cergy Pontoise(FR)**
Inventeur: **Martin, Jean-Paul**
**3bis Rue Victor Hugo**
**F-92700 Colombes(FR)**
Inventeur: **Renault, Christian**
**61 Rue des Mallets**
**F-95150 Taverny(FR)**

(74) Mandataire: **Savina, Jacques et al**
**RHONE-POULENC SANTE, Service Brevets,**
**20 Avenue Raymond Aron**
**F-92165 Antony Cédex(FR)**

## (54) Nouveaux dérivés du benzopyranne, leur preparation et les compositions pharmaceutiques qui les contiennent.

(57) Nouveaux dérivés du benzopyranne de formule générale (I) dans laquelle :
- $R_1$ représente un atome d'hydrogène ou d'halogène ou un radical hydroxy, alcoyloxy, nitro, amino, alcoylsulfonamido ou acylamino,
- R représente
1) un radical

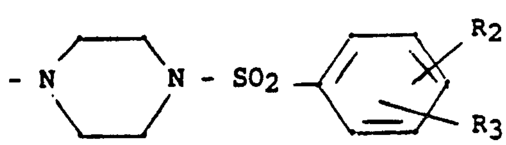

$R_2$ et $R_3$ identiques ou différents étant H, halogène, OH, alcoyle, alcoyloxy, $NH_2$, alcoylsulfonamido ou $NO_2$, ou
2) un radical

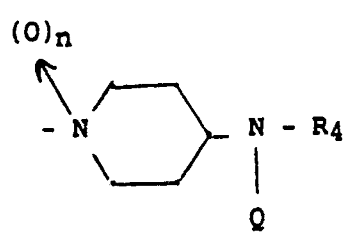

EP 0 379 441 A1

n étant 0 ou 1, $R_4$ étant H, alcoyle ou phényle éventuellement substitué et Q est acyle, alcoylsulfonyle, ou

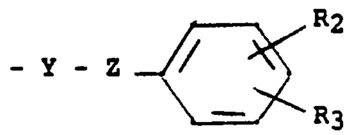

Y étant -CO- ou $-SO_2-$ et Z étant une liaison simple, $-CH_2-$ ou -NH-, ou

3) un radical

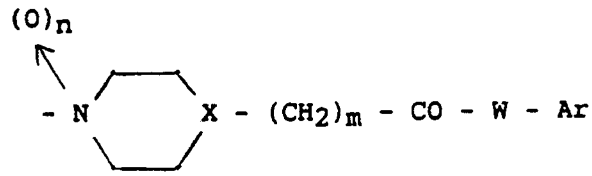

n étant 0 ou 1, m étant 0 ou 2, X étant C ou N (si n = 0), W étant une liaison ou -NH- et Ar étant pyridyle, indolyle, quinolyle, alcoyl-2 quinolyle ou phényle éventuellement substitué par $R_2$ et $R_3$, à condition que n = O lorsque X est N ou

4) un radical de formule générale :

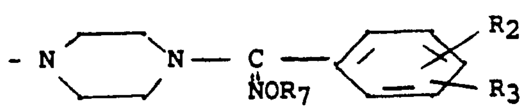

$R_7$ étant H ou alcoyle, ou bien

5) un radical

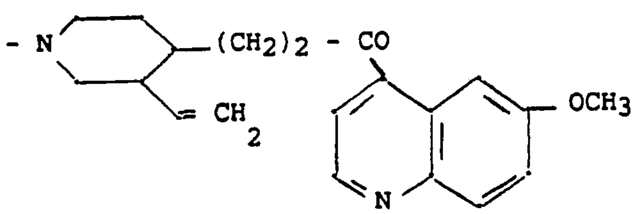

- $R'$ et $R''$ sont identiques et représentent des atomes d'hydrogène ou des radicaux alcoyle,leurs formes isomères et leurs mélanges, leurs sels d'addition avec les acides.

Ces nouveaux produits sont utiles comme agents antiarythmiques et antifibrillants.

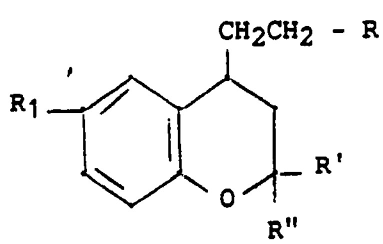

(I)

# NOUVEAUX DERIVES DU BENZOPYRANNE, LEUR PREPARATION ET LES COMPOSITIONS PHARMACEUTIQUES QUI LES CONTIENNENT

La présente invention concerne de nouveaux dérivés du benzopyranne de formule générale :

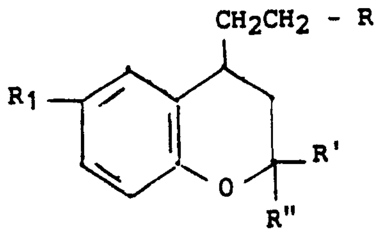

(I)

leurs sels, leur préparation et les compositions pharmaceutiques qui les contiennent.

Dans la demande de brevet allemand 3 300 004 ont été décrits des dérivés de l'aminométhyl-4 benzopyranne actifs comme hypotenseurs et relaxants musculaires, et répondant à la formule :

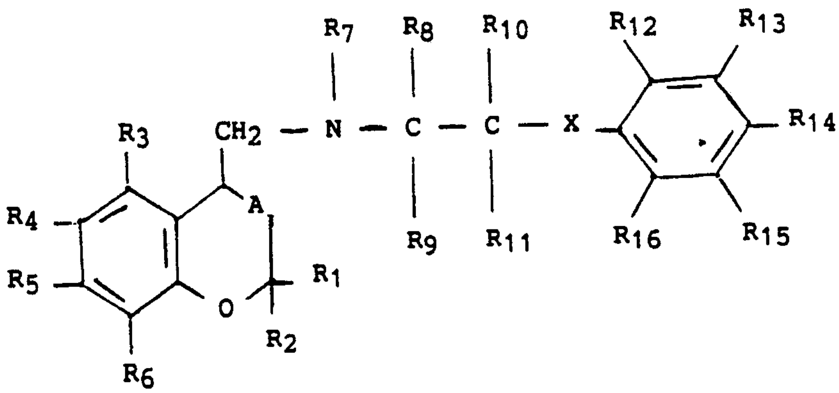

dans laquelle

- A représente notamment une liaison simple,
- $R_1$, $R_2$, $R_8$, $R_9$, $R_{10}$ et $R_{11}$ peuvent représenter des atomes d'hydrogène,
- $R_3$, $R_4$, $R_5$ et $R_6$ peuvent être des atomes d'hydrogène ou des radicaux alcoyloxy,
- $R_{12}$ à $R_{16}$ peuvent être entre autres des atomes d'hydrogène, des radicaux alcoyloxy ou 2 de ces radicaux adjacents peuvent former un radical méthylènedioxy,
- et $-NR_7-CR_8R_9-CR_{10}R_{11}-X-$ peut représenter un radical pipérazinyle.

Il a été trouvé que les produits de formule générale (I) dans laquelle

- $R_1$ représente un atome d'hydrogène ou d'halogène ou un radical hydroxy, alcoyloxy, nitro, amino, alcoylsulfonamido ou acylamino,
- R représente

1) un radical de formule générale :

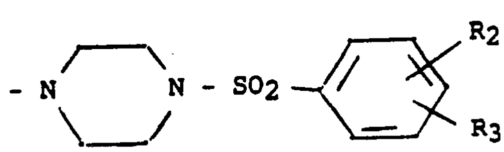

(II)

dans laquelle $R_2$ et $R_3$ identiques ou différents représentent des atomes d'hydrogène ou d'halogène ou des radicaux hydroxy, alcoyle, alcoyloxy, amino, alcoylsulfonamido ou nitro, ou bien

2) un radical de formule générale :

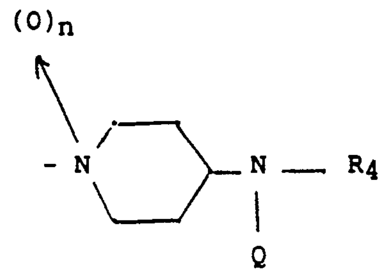

(III)

dans laquelle n égale 0 ou 1, $R_4$ est un atome d'hydrogène, un radical alcoyle ou un radical de structure :

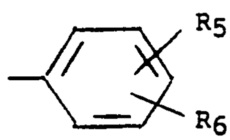

(IVa)

dans laquelle $R_5$ et $R_6$ sont des atomes d'hydrogène ou d'halogène ou un radical alcoyloxy et Q représente un radical acyle, alcoylsulfonyle ou un radical de structure :

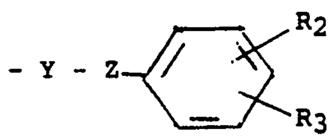

(IVb)

dans laquelle $R_2$ et $R_3$ sont définis comme ci-dessus, Y représente un radical carbonyle ou sulfonyle et Z représente une liaison simple ou un radical méthylène ou imino, ou bien

3) un radical de formule générale :

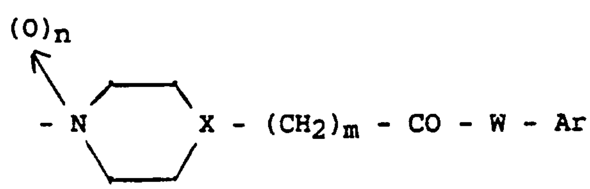

(V)

dans laquelle n égale 0 ou 1, m égale 0 à 2, X est un atome de carbone ou X peut être un atome d'azote si n = 0, W représente une liaison simple ou un radical imino et Ar représente un radical pyridyle, indolyle, quinolyle, alcoyl-2 quinolyle ou Ar représente un radical phényle éventuellement substitué par des radicaux $R_2$ et $R_3$ tels que définis ci-dessus, à condition que m soit autre que O lorsque X est un atome d'azote, ou bien

4) un radical de formule générale :

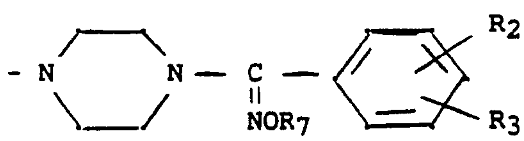

(VI)

dans laquelle $R_2$ et $R_3$ sont définis comme précédemment et $R_7$ représente un atome d'hydrogène ou un radical alcoyle, ou bien

5) un radical de formule :

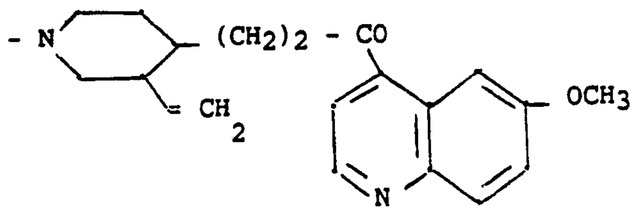

(VII)

- R′ et R″ identiques représentent des atomes d'hydrogène ou des radicaux alcoyle, ainsi que leurs sels, entraînent notamment une augmentation des périodes réfractaires particulièrement intéressante qui correspond aux effets antifibrillants des produits antiarythmiques de la classe III selon la classification de VAUGHAN WILLIAMS.

Dans la formule générale (I), lorsque $R_1$ et/ou $R_2$, $R_3$, $R_5$ ou $R_6$ (dans le symbole R) représentent un atome d'halogène, celui-ci peut être choisi parmi le fluor, le chlore, le brome ou l'iode. De plus, il est entendu que les radicaux et portions alcoyle ou acyle peuvent être droits ou ramifiés et contiennent 1 à 4 atomes de carbone.

Il est également entendu que les produits de formule générale (I) présentent des formes isomères et que ces isomères et leurs mélanges entrent dans le cadre de la présente invention.

Selon l'invention, les produits de formule générale (I) peuvent être obtenus par action d'un produit de formule générale :

H - R (VIII)

ou de son sel, dans laquelle R est défini comme précédemment à condition que n = 0, sur un dérivé de benzopyranne de formule générale :

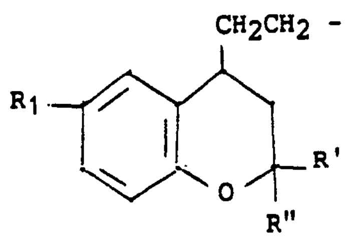

(IX)

dans laquelle $R_1$, R′ et R″ sont définis comme précédemment et $Y_1$ représente un atome d'halogène ou un radical alcoylsulfonyloxy ou arylsulfonyloxy suivie éventuellement de l'oxydation du produit obtenu, lorsque l'on veut préparer un produit pour lequel n = 1, ou bien de la transformation en oxime lorsque l'on veut obtenir un produit pour lequel R est un radical défini précédemment en 4) et lorsque l'on a obtenu la cétone correspondante pour laquelle R est un radical défini précédemment en 3) m étant égal à O et - W - Ar étant un radical phényle éventuellement substitué.

On opère avantageusement en présence d'un agent accepteur d'acide. Il est également possible d'opérer sans accepteur d'acide, en présence de 2 équivalents du produit de formule générale (VIII).

Lorsque $Y_1$ représente un atome d'halogène, il peut être choisi parmi les atomes de chlore ou de brome.

Lorsque $Y_1$ représente un radical alcoylsulfonyloxy, il représente notamment le radical méthylsulfonyloxy et lorsqu'il représente un radical arylsulfonyloxy, il peut être entre autres le radical p.toluènesulfonyloxy.

A titre d'accepteur d'acide, on utilise avantageusement un hydroxyde de métal alcalin ou alcalinoterreux (soude ou potasse par exemple), un carbonate de métal alcalin (bicarbonate de sodium, carbonate de potassium par exemple), ou une base organique azotée telle que la triéthylamine par exemple.

La réaction s'effectue dans un solvant inerte tel qu'une cétone (acétone, butanone par exemple), un éther (tétrahydrofuranne ou dioxanne par exemple), un alcool (méthanol ou éthanol par exemple), un hydrocarbure (hexane ou toluène par exemple), l'acétonitrile, le diméthylformamide ou le diméthylsulfoxyde, ou dans un mélange de tels solvants, à une température comprise entre 20 °C et la température de reflux du mélange réactionnel.

Il est entendu que dans les cas où $R_1$, $R_2$ et/ou $R_3$ (dans R) représentent un radical amino, ce dernier est préalablement protégé. De même lorsque $R_2$ et/ou $R_3$ représentent un radical hydroxy, il est préférable de protéger ce radical préalablement à la réaction.

La protection s'effectue par tout groupement compatible et dont la mise en oeuvre et l'élimination

n'altèrent pas le reste de la molécule. Notamment on opère selon les méthodes décrites par T.W. Greene, Protective Groups in Organic Synthesis, A. Wiley -Interscience Publication (1981), ou par Mc Omie, Protective Groups in Organic Chemistry, Plenum Press (1973).

Le cas échéant, l'oxydation s'effectue par toute méthode connue qui n'altère pas le reste de la molécule. La réaction s'effectue notamment au moyen d'un agent oxydant tel qu'un péracide organique, par exemple l'acide péracétique ou l'acide monoperphtalique, dans un solvant organique tel qu'un éther (éther éthylique, tétrahydrofuranne par exemple) ou un solvant chloré (chloroforme, dichlorométhane par exemple) à une température comprise entre 0 et 25° C. L'oxydation peut également être effectuée au moyen de l'eau oxygénée en opérant en milieu aqueux ou dans l'acide acétique ou l'anhydride acétique à une température comprise entre -50 et +25° C.

Il est entendu que, dans le cas où la molécule porte des substituants amino, l'oxydation est effectuée avant la libération des radicaux protecteurs.

Le cas échéant, la transformation en un produit pour lequel R est un radical de formule générale (VI) dans laquelle $R_7$ est un atome d'hydrogène ou un radical alcoyle, s'effectue par action du chlorhydrate d'hydroxylamine ou d'0-alcoylhydroxylamine.

On opère généralement en présence d'une base (soude par exemple) dans un alcool (éthanol absolu) à une température comprise entre 20 et 80° C.

Selon l'invention, les produits de formule générale (I) pour lesquels les radicaux $R_1$, $R_2$ et/ou $R_3$ représentent un radical hydroxy peuvent également être obtenus à partir du produit de formule générale (I) correspondant pour lequel le radical $R_1$, $R_2$ et/ou $R_3$ à transformer représente un radical alcoyloxy, par traitement en milieu acide concentré.

La réaction s'effectue généralement par traitement par l'acide bromhydrique, ou un mélange d'acides, par exemple par traitement par un mélange acide bromhydrique - acide acétique, à la température de reflux du mélange réactionnel.

Selon l'invention, les produits de formule générale (I) pour lesquels R est défini comme précédemment en 1) et 2), et les symboles $R_1$, $R_2$ et/ou $R_3$ représentent un radical amino ou alcoylsulfonamido ou pour lesquels $R_1$ représente un radical acylamino, peuvent également être obtenus par hydrogénation catalytique en milieu acide du dérivé correspondant du benzopyranne de formule générale (I) pour lequel le radical $R_1$, $R_2$ et/ou $R_3$ à transformer représente un radical nitro, puis lorsque l'on veut obtenir un produit de formule générale (I) pour lequel $R_1$, $R_2$ et/ou $R_3$ repré sentent un radical alcoylsulfonamido ou pour lequel $R_1$ est un radical acylamino, on transforme le dérivé aminé obtenu respectivement par sulfonylation ou par acylation.

L'hydrogénation s'effectue avantageusement à une température comprise entre 20 et 50° C, dans un acide comme par exemple l'acide acétique ou l'acide chlorhydrique, dans un solvant organique tel qu'un alcool (méthanol, éthanol, isopropanol par exemple) dans un mélange de solvants, ou en milieu hydroorganique (alcool - eau par exemple). Il est également possible d'opérer directement dans l'acide, sans addition supplémentaire d'un solvant.

A titre de catalyseur, on utilise généralement le palladium, l'oxyde de platine ou le nickel de Raney.

Eventuellement on opère sous pression.

La sulfonylation ou l'acylation s'effectuent respectivement par action d'une forme activée d'un acide $alkSO_3H$ ou alk′COOH (alk et alk′ étant des radicaux alcoyle), notamment l'halogénure d'acide (chlorure d'acide par exemple) ou l'anhydride, et l'on opère en présence d'un accepteur d'acide tel qu'une base organique azotée comme une trialcoylamine (triéthylamine par exemple) ou comme la pyridine, dans un solvant organique inerte tel qu'un solvant chloré (dichlorométhane, chloroforme par exemple), un éther (éther éthylique, tétrahydrofuranne par exemple) ou dans un mélange de ces solvants, à une température comprise entre -70 et +40° C.

Eventuellement on opère sous azote.

Selon l'invention, les produits de formule générale (I) pour lesquels R est défini comme précédemment en 1) et 3) lorsque X est un atome d'azote, peuvent aussi être préparés par action d'un halogénure de structure :

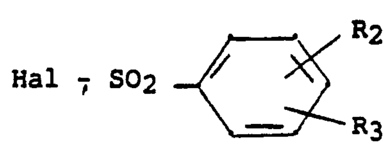 (Xa)

ou $Hal - (CH_2)_m - CO - W - Ar$ (Xb)

dans laquelle $R_2$, $R_3$, W, Ar et m sont définis comme précédemment, et Hal est un atome d'halogène choisi parmi le chlore ou le brome sur un dérivé du benzopyranne de formule générale :

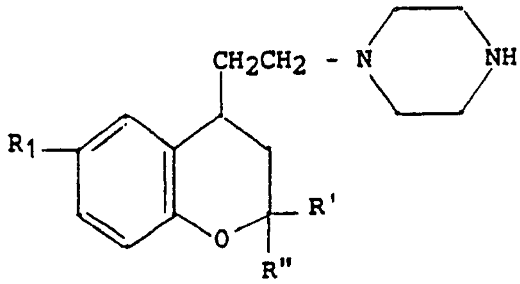

(XI)

dans laquelle $R_1$, $R'$ et $R''$ sont définis comme précédemment.

Il est entendu que, lorsque $R_1$, $R_2$ et/ou $R_3$ représentent des radicaux amino ou hydroxyl ceux-ci sont protégés préalablement à la réaction.

La protection et l'élimination des radicaux protecteurs s'effectue dans les conditions décrites précédemment pour le procédé qui consiste à faire réagir les produits de formule générale (VIII) et (IX).

Lorsque l'on fait agir un produit de formule générale (Xa) ou (Xb) dans lequel m = 0, on opère soit en milieu organique, éventuellement en présence d'un accepteur d'acide tel qu'une base organique azotée (par exemple une trialcoylamine ou une pyridine) dans un solvant tel que cité ci-dessus, ou un mélange de ces solvants à une température comprise entre 0 et 20 °C, soit en milieu hydroorganique en présence d'un agent alcalin de condensation tel qu'un carbonate ou un bicarbonate de métal alcalin ou alcalino terreux, à une température comprise entre 5 et 20 °C.

Lorsque l'on fait agir un produit de formule générale (Xb) pour lequel m est 1 ou 2, on opère dans les conditions décrites précédemment pour préparer un produit de formule générale (I) à partir des produits de formules générales (VIII) et (IX).

Les produits de formule générale (VIII) peuvent être préparés :

- lorsque R est défini comme précédemment en 1) ou 3), X étant un atome d'azote : par action d'un dérivé halogéné de formule (Xa) ou (IXb) sur la pipérazine dans les conditions décrites précédemment pour la réaction des produits de formule générale (VIII) avec un dérivé du benzopyranne de formule générale (IX) en présence d'un excès de pipérazine, sans addition supplémentaire d'un accepteur d'acide ;
- lorsque R est défini comme en 2) : selon la méthode décrite dans le brevet français M 2430, ou selon les méthodes mentionnées par Anwer Basha, Tet. Lett., 29(21), 2525 (1988) ;
- lorsque R est défini comme en 3), X étant un atome d'azote et W un radical imino : à partir d'une benzylpipérazine, par application des méthodes mentionnées par Anwer Basha, Tet. Lett., 29(21), 2525 (1988) puis de l'élimination du radical protecteur de la pipérazine;
- lorsque R est défini comme en 3), X étant un atome de carbone,

a) si W est une liaison : par réaction de Friedel et Crafts entre un chlorure d'acide de formule générale :

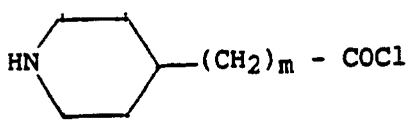

(XII)

dans laquelle m égale 1 ou 2, et un produit de formule générale :

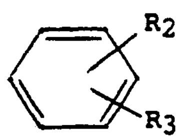

(XIII)

dans laquelle $R_2$ et $R_3$ sont identiques ou différents et représentent des atomes d'hydrogène, d'halogène ou un radical amino préalablement protégé, ou par application de la méthode décrite par P. Rabbe, Ber., 55, 532 (1922) ;

b) si W est un radical imino : par application de la méthode décrite par L.D. Wise et coll., J. Med. Chem., 28, 606 (1985) ;

- lorsque R est défini comme en 5) : selon la méthode décrite par A. Quevauviller et coll., Ann. Pharm. Franc., 24, 39 (1966).

Les produits de formule générale (IX) peuvent être obtenus par action d'un agent d'halogénation ou d'une forme activée d'un acide alcoylsulfonique ou arylsulfonique sur un dérivé de l'hydroxyalcoyl-4 benzopyranne de formule générale :

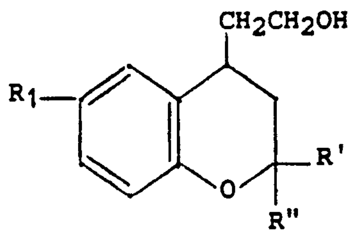

(XIV)

dans laquelle $R_1$, $R'$ et $R''$ sont définis comme précédemment.

Lorsque l'on veut préparer un produit de formule générale (IX) pour lequel $Y_1$ est un atome d'halogène, les agents d'halogénation peuvent être choisis parmi le chlorure de thionyle ou les dérivés halogénés du phosphore, tels que l'oxychlorure de phosphore ou le tribromure de phosphore. Il est également possible de faire réagir le bromure d'allyle en présence de NN'-carbonyldiimidazole.

Lorsque l'on veut préparer un produit de formule générale (IX) dans laquelle $Y_1$ est alcoylsulfonyloxy ou arylsulfonyloxy, on fait avantageusement réagir l'anhydride ou l'halogénure de l'acide correspondant.

La réaction s'effectue généralement en présence d'une base organique azotée telle que la triéthylamine ou la pyridine, dans un solvant organique tel qu'un solvant chloré (chlorure de méthylène par exemple), un éther (tétrahydrofuranne, dioxanne par exemple), en opérant à une température comprise entre 0° C et la température de reflux du mélange réactionnel.

Les produits de formule générale (IX) dans laquelle $R_1$ est un radical nitro peuvent être obtenus par nitration d'un dérivé de formule générale (IX) pour lequel $R_1$ est un atome d'hydrogène.

On opère avantageusement au moyen du mélange acide nitrique - acide acétique à une température comprise entre 0 et 20° C.

Les produits de formule générale (IX) dans laquelle $R_1$ est un radical hydroxy peuvent également être obtenus à partir d'un produit de formule générale (IX) dans laquelle $R_1$ est un radical alcoyloxy, par traitement en milieu acide concentré. On opère dans les conditions décrites précédemment pour l'obtention d'un produit de formule générale (I) pour lequel $R_1$ représente un radical hydroxy à partir du produit correspondant pour lequel $R_1$ est un radical alcoyloxy.

Le dérivé de l'hydroxyalcoyl-4 benzopyranne de formule générale (XIV) peut être préparé par réduction de l'ester correspondant de formule générale :

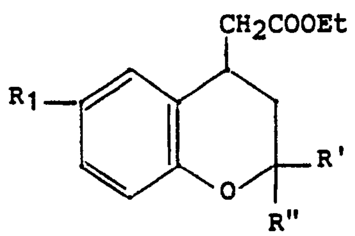

(XV)

dans laquelle $R_1$, $R'$ et $R''$ sont définis comme précédemment.

On opère généralement au moyen d'hydrure d'aluminium et de lithium dans un solvant organique tel qu'un éther (tétrahydrofuranne par exemple) à une température comprise entre 0 et 30° C.

L'ester de formule générale (XVII) peut être obtenu par réduction du dérivé du benzopyranne de formule générale :

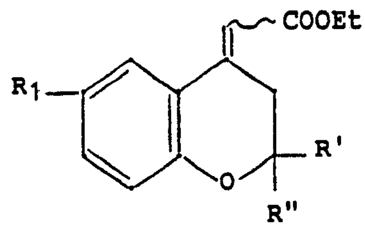

(XVI)

dans laquelle $R_1$, $R'$ et $R''$ sont définis comme précédemment.

On opère par hydrogénation catalytique en présence de palladium, dans un solvant organique tel qu'un alcool (méthanol, éthanol par exemple), à une température comprise entre 10 et 50 °C.

Le dérivé du benzopyranne de formule générale (XVI) peut être préparé par réaction de WITTIG, à partir d'un dérivé de la chromannone-4 de formule générale :

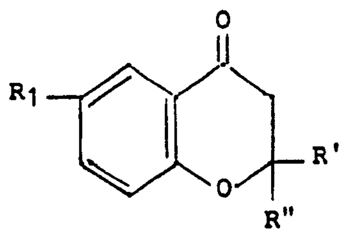

(XVII)

dans laquelle $R_1$, $R'$ et $R''$ sont définis comme précédemment.

On opère avantageusement au moyen de diéthylphosphonoacétate d'éthyle en présence d'hydrure de sodium, dans un solvant organique tel qu'un éther (tétrahydrofuranne ou diméthoxyéthane par exemple) à une température comprise entre 0 °C et la température de reflux du mélange réactionnel.

Le dérivé de la chromannone-4 de formule générale (XVII) dans laquelle $R_1$ est autre que l'hydrogène, peut être préparé par application de la méthode décrite par PFEIFFER et coll., Chem. Ber., 58 (1954), ou selon les méthodes décrites par G.P. Ellis, Heterocyclic compounds, chromenes, chromanones and chromones, John Wiley and Sons (1977).

Le dérivé de la chromannone-4 de formule générale (XVII) dans laquelle $R_1$ est un atome de fluor, peut être préparé selon la méthode décrite dans la demande de brevet français 2 588 860.

Les dérivés de la chromannone-4 de formule générale (XVII) dans laquelle $R_1$ est un radical amino, alcoylsulfonamido ou acylamino, peuvent être obtenus à partir du dérivé de la chromannone-4 de formule générale (XVII) pour lequel $R_1$ est un radical nitro, par analogie avec les méthodes décrites pour la préparation des produits de formule générale (I) pour lesquels le radical $R_1$ est défini comme ci-dessus.

La diméthyl-2,2 chromannone-4 peut être obtenue selon la méthode décrite dans le brevet belge 844 943.

Les produits de formule générale (Xb) peuvent être préparés

- lorsque W est une liaison, par réaction de Friedel et Crafts entre un chlorure d'acide de formule générale :

$Hal(CH_2)_mCOCl$ (XVIII)

dans laquelle m est égale à 0 ou 1 et un produit de formule générale (XIII), ou

- lorsque W est un radical imino, selon la méthode décrite par L.D. Wise et coll., J. Med. Chem., 28, 606 (1985).

Les dérivés du benzopyranne de formule générale (XI) peu vent être obtenus par action de la pipérazine sur un dérivé du benzopyranne de formule générale (IX).

La réaction s'effectue dans les conditions décrites précédemment pour la réaction des produits de formule générale (VIII) avec les dérivés du benzopyranne de formule générale (IX), en présence d'un excès de pipérazine (2 équivalents), sans addition supplémentaire d'un accepteur d'acide.

Les énantiomères des produits selon l'invention peuvent être séparés selon les méthodes connues.

On opère notamment par préparation de l'énantiomère du dérivé de l'hydroxyéthylbenzopyranne de formule générale (XIV) qui est transformé en produit de formule générale (I) selon le procédé décrit précédemment.

Le dérivé optiquement actif de formule générale (XIV) est obtenu par préparation d'un amide optiquement actif de formule générale :

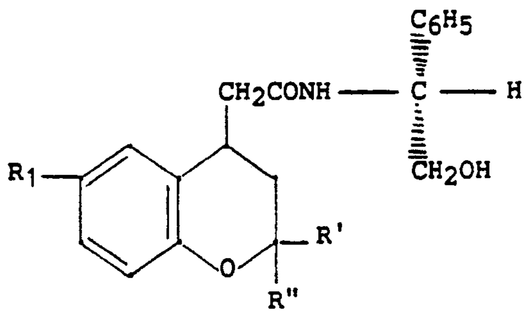

(XIX)

dans laquelle $R_1$, R′ et R″ sont définis comme précédemment, séparation des isomères par chromatographie, hydrolyse de l'isomère recherché puis réduction de l'acide obtenu.

L'hydrolyse de l'isomère du produit de formule générale (XIX) peut être effectuée par toute méthode connue qui n'altère pas le reste de la molécule ; on opère avantageusement en milieu acide (acide acétique, acide chlorhydrique en mélanges) à la température de reflux du mélange réactionnel.

La réduction de l'acide en alcool est mise en oeuvre selon les méthodes habituelles. Notamment on utilise le diboranne à titre d'agent réducteur et l'on opère avantageusement dans un éther tel que le tétrahydrofuranne à des températures comprises entre 0 et 30 °C.

Le produit de formule générale (XIX) peut être préparé à partir de l'acide de formule générale :

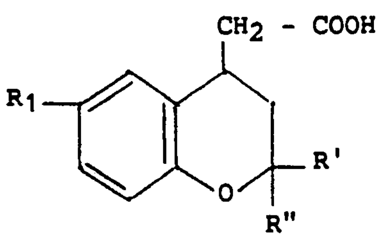

(XX)

dans laquelle $R_1$, R′ et R″ sont définis comme précédemment, par toute méthode connue pour préparer un amide à partir d'un acide.

On opère avantageusement au moyen du chlorure de l'acide de formule générale (XX) (qui peut être préparé in situ) dans un solvant organique inerte tel qu'un solvant chloré (dichlorométhane par exemple) en présence d'un agent accepteur d'acide comme une base organique azotée (triéthylamine par exemple), à une température comprise entre 0 et 30 °C.

L'acide de formule générale (XX) peut être obtenu à partir de l'ester correspondant, par toute méthode connue pour obtenir un acide à partir d'un ester sans toucher au reste de la molécule.

On effectue notamment la saponification de l'ester de formule générale (XIV) par la potasse, dans le méthanol à la température de reflux du mélange réactionnel.

Le chlorure d'acide est préparé par traitement de l'acide correspondant par le chlorure de thionyle à la température de reflux du mélange réactionnel.

Les nouveaux dérivés du benzopyranne selon l'invention peuvent être purifiés le cas échéant, par des méthodes physiques telles que la cristallisation ou la chromatographie.

Les produits selon l'invention peuvent être transformés en sels d'addition avec les acides. Le sel formé précipite après concentration éventuelle de sa solution, il est séparé par filtration, décantation ou lyophilisation. Selon le procédé de la présente invention, les produits sont généralement obtenus à l'état de d'oxalate. Ces sels peuvent être libérés et transformés en sels d'autres acides selon les méthodes habituelles.

Comme exemples de sels pharmaceutiquement acceptables peuvent être cités les sels d'addition avec des acides minéraux (chlorhydrates, bromhydrates, sulfates, nitrates, phosphates) ou organiques (succinates, fumarates, acétates, propionates, maléates, méthanesulfonates, p.toluènesulfonates, iséthionates) ou des dérivés de substitution de ces composés.

Les produits selon l'invention manifestent des propriétés antiarythmiques. Notamment leurs propriétés antifibrillantes particulièrement intéressantes, caractéristiques de la classe III de VAUGHAN WILLIAMS, se traduisent par un allongement des périodes réfractaires.

Ils provoquent, in vitro sur muscle papillaire de cobaye, une augmentation comprise entre 5 % et des

valeurs supérieures à 30 %, de la durée du potentiel d'action initial, selon la technique des mesures d'enregistrement du potentiel d'action intracellulaire décrite par E. CORABOEUF et S. WEIDMANN, C.R. Soc. Biol., 143, 1329 (1949).

Par ailleurs, les dérivés du benzopyranne selon l'invention manifestent une faible toxicité. Ils se sont généralement montrés atoxiques à 300 mg/kg par voie orale chez la souris.

D'un intérêt particulier sont les produits de formule générale (I) pour lesquels,
$R_1$, $R'$ et $R''$ sont des atomes d'hydrogène, et R représente un radical tel que défini en 1) pour lequel $R_2$ et $R_3$ sont des atomes d'hydrogène, ou
R représente un radical tel que défini en 2) pour lequel n égale O, $R_4$ est un atome d'hydrogène, un radical alcoyle ou un radical de structure (IVa) dans laquelle $R_5$ est $R_6$ sont des atomes d'hydrogène ou d'halogène, ou des radicaux alcoyloxy et Q représente un radical acétyle, méthylsulfonyle ou un radical de formule générale (IVb) dans laquelle Y est un radical carbonyle ou sulfonyle et Z est une liaison ou un radical méthylène ou imino, et $R_2$ et $R_3$ sont des atomes d'hydrogène ou d'halogène ou des radicaux alcoyle ou méthylsulfonamido, ou
R représente un radical tel que défini en 3) pour lequel n égale O, m égale 0 à 2, W est une liaison ou un radical imino, Ar est pyridyle, indolyle ou phényle éventuellement substitué par un atome d'halogène, ou des radicaux alcoyle ou alcoyloxy et X est un atome de carbone ou d'azote, ou R représente un radical tel que défini en 4) pour lequel $R_7$ représente un atome d'hydrogène.

Et parmi ces produits plus spécialement actifs sont les produits de formule générale (I) donnés ci-après :

- N-{[dihydro-3,4 2H-benzopyran-1 yl-4)-2 éthyl]-1 pipéridyl-4} acétanilide et ses sels ;
- [(dihydro-3,4 2H-benzopyran-1 yl-4)-2 éthyl]-1 nicotinoyl-4 pipérazine et ses sels ;
- N-{[dihydro-3,4 2H-benzopyran-1 yl-4)-2 éthyl}-1 pipéridyl-4} N-phényl fluoro-4 benzamide et ses sels ;
- benzoyl-4 [(dihydro-3,4 2H-benzopyran-1 yl-4)-2 éthyl]-1 pipéridine et ses sels ;
- {[dihydro-3,4 2H-benzopyran-1 yl-4)-2 éthyl]-1 pipéridyl-4} phényl méthanoxime et ses sels ;

Les exemples suivants donnés à titre non limitatif illustrent la présente invention.

Dans les exemples qui suivent, sauf mention spéciale, les chromatographies sont mises en oeuvre sur gel de silice (60-200 μ).

## EXEMPLE 1

On chauffe à reflux pendant 6 heures, 1,56 g de (bromo-2 éthyl)-4 dihydro-3,4 2H-benzopyranne, 2 g de N-(pipéridyl-4) diméthoxy-3,4 acétanilide, 1,6 g de carbonate de potassium sec et 1,07 g d'iodure de potassium dans 50 $cm^3$ de butanone-2.

On filtre le mélange réactionnel sur verre fritté puis après évaporation du solvant sous pression réduite (5,2 kPa), on reprend l'huile obtenue par 10 cm3 d'une solution (5N) de carbonate de potassium et ajoute 40 cm3 d'eau puis on extrait par deux fois 75 cm3 d'acétate d'éthyle.

La phase organique est ensuite séchée sur sulfate de magnésium puis concentrée à sec sous pression réduite.

Le résidu d'évaporation est chromatographié sur une colonne de 2,8 cm de diamètre contenant 25 g de gel de silice en éluant par 210 $cm^3$ d'un mélange dichlorométhane - isopropanol (90-10 en volumes) et en recueillant des fractions de 30 $cm^3$. Les fractions comprises entre 60 et 210 $cm^3$ sont concentrées à sec.

On reprend l'huile obtenue dans le minimum d'acétone et ajoute 0,39 g d'acide oxalique dissous dans l'acétone puis on concentre à sec et cristallise dans la butanone-2.

On obtient ainsi 1,6 g d'oxalate acide de N-{[(dihydro-3,4 2H-benzopyran-1 yl-4)-2 éthyl]-1 pipéridyl-4} diméthoxy-3,4 acétanilide sous forme d'un solide blanc fondant à 174° C.

Le N-(pipéridyl-4) diméthoxy-3,4 acétanilide peut être préparé par application de la méthode décrite dans le brevet français M 2430, mais à partir de 8,4 g de N-(benzyl-1 pipéridyl-4) diméthoxy-3,4 acétanilide, de 0,85 g de palladium sur charbon (5 %) dans 100 cm3 d'un mélange acide acétique - eau (70-30 en volumes) et en ef fectuant l'hydrogénation à 60° C.

On obtient ainsi 6,3 g de N-(pipéridyl-4) diméthoxy-3,4 acétanilide sous forme d'une poudre blanchâtre fondant à 210° C.

Le N-(benzyl-1 pipéridyl-4) diméthoxy-3,4 acétanilide peut être préparé selon la méthode décrite dans le brevet français M 2430, mais à partir de 10 g de N-benzyl (diméthoxy-3,4 anilino)-4 pipéridine, 20 cm3 d'anhydride acétique dans 30 cm3 d'acide acétique.

On obtient ainsi 10,9 g de N-(benzyl-1 pipéridyl-4) diméthoxy-3,4 acétanilide sous forme d'un solide blanchâtre fondant à 117° C.

La N-benzyl (diméthoxy-3,4 anilino)-4 pipéridine peut être préparée selon la méthode décrite dans le brevet français M 2430, mais à partir de 37,8 g de N-benzylpipéridone, de 19,2 g de diméthoxy-3,4 aniline dans 200 cm3 de toluène additionnés de 0,017 g d'acide paratoluènesulfonique et de 1,4 g d'hydrure de lithium et d'aluminium dans 200 cm3 d'éther éthylique.

On obtient ainsi 15 g de N-benzyl (diméthoxy-3,4 anilino)-4 pipéridine sous forme d'un solide blanchâtre fondant à 66° C.

Le bromo-2 éthyl-4 dihydro-3,4 2H-benzopyranne peut être préparé de la manière suivante :

A 115 $cm^3$ d'acétonitrile on ajoute, sous agitation, 13,8 g de (dihydro-3,4 2H-benzopyran-1 yl-4)-2 éthanol, puis 91,2 g de bromure d'allyle et enfin 12,6 g de NN'-carbonyldiimidazole.

On agite 3 heures 10 minutes à 20° C environ puis 2 heures à reflux.

Le mélange réactionnel est ensuite concentré sous pression réduite (5,2 kPa) et le résidu obtenu est chromatographié sur une colonne de 5,5 cm de diamètre contenant 200 g de gel de silice en éluant par 550 $cm^3$ de dichlorométhane et en recueillant des fractions de 100 $cm^3$. Les fractions comprises entre 350 et 550 $cm^3$ sont concentrées à sec.

On obtient ainsi 17,7 g de (bromo-2 éthyl)-4 dihydro-3,4 2H-benzopyranne sous forme d'une huile brun clair.

Spectre de RMN du proton (250 MHz, $CDCl_3$, $\delta$ en ppm) :

6,8 à 7,2 (mt, 4H aromatiques)
4,21 (mt, $-O-CH_2-$)
3,55 (mt, $-CH_2-Br$)
3,08 (mt, $\geq CH-$)
1,92 et 2,92 (mt, $-CH_2-$ en -3)
2,08 et 2,34 (mt, $-CH_2CH_2Br$)

Le (dihydro-3,4 2H-benzopyran-1 yl-4)-2 éthanol peut être préparé de la manière suivante :

A 5,96 g d'hydrure de lithium et d'aluminium, on ajoute 500 $cm^3$ de tétrahydrofuranne et refroidit à 0° C. On ajoute alors, sous agitation, 17,25 g de (dihydro-3,4 2H-benzopyran-1 yl-4)-2 éthanoate d'éthyle dans 60 $cm^3$ de tétrahydrofuranne.

Après 1 heure d'agitation à 20° C, on hydrolyse sous agitation par addition de sulfate de sodium hydraté (10 $H_2O$) jusqu'à précipitation puis on laisse reposer le mélange réactionnel pendant heures.

Après filtration du précipité formé et évaporation du solvant sous pression réduite, on isole 13,8 g de (dihydro-3,4 2H-benzopyran-1 yl-4)-2 éthanol sous forme d'une huile brune.

Spectre de RMN (250 MHz, $CDCl_3$, $\delta$ en ppm) :

6,8 à 7,2 (mt, 4H aromatiques)
4,22 (mt, $-O-CH_2-$)
3,83 (mt, $-CH_2-OH$)
3,04 (mt, $\geq CH-$)
1,83 et 2,90 (mt, $-CH_2-$ en -3 et $-CH_2-CH_2OH$)
1,62 (s, -OH)

Le (dihydro-3,4 2H-benzopyran-1 yl-4) éthanoate d'éthyle peut être préparé de la manière suivante :

50,6 g de (dihydro-3,4 2H-benzopyran-1 ylidène-4) acétate d'éthyle (E,Z) dans 1 litre de méthanol, sont hydrogénés à 20° C sous pression atmosphérique, en présence de 5,06 g de palladium sur charbon (10 %).

Après filtration sur KIESELGUHR et concentration à sec sous pression réduite (5,2 kPa), on obtient 48,8 g de (dihydro-3,4 2H-benzopyran-1 yl-4) éthanoate d'éthyle sous forme d'une huile jaune pâle.

Spectre de RMN (250 MHz, $CDCl_3$, $\delta$ en ppm) :

6,75 à 7,2 (mt, 4H aromatiques)
4,98 (q + mt, $-O-CH_2-$ + $-CO-OCH_2-CH_3$)
3,37 (mt, $\geq CH-$)
2,53 et 2,82 (dd, $-CH_2-CO-$)
1,87 et 2,18 (mt, $-CH_2-$ en -3)
1,30 (t, $-COO-CH_2-CH_3$)

Le (dihydro-3,4 2H-benzopyran-1 ylidène-4) acétate d'éthyle (E,Z) peut être préparé de la manière suivante :

A 1 litre de tétrahydrofuranne anhydre, on ajoute, sous agitation, 20,4 g d'hydrure de sodium (80 %) puis par petites portions 153 g de diéthylphosphonoacétate d'éthyle tout en maintenant la température du mélange réactionnel aux environs de 20° C. Puis la solution jaune claire ainsi obtenue est additionnée de 45 g de chromannone-4 dans 100 $cm^3$ de tétrahydrofuranne anhydre en maintenant la température en dessous de 0° C. Après 22 heures à 20° C, on concentre sous pression réduite le mélange réactionnel puis extrait

l'huile obtenue par 2 fois 700 $cm^3$ de dichlorométhane. La phase organique est lavée à l'eau puis séchée sur sulfate de magnésium et concentrée à sec sous pression réduite. Le résidu d'évaporation est chromatographié sur une colonne de 9 cm de diamètre, contenant 1,6 kg de gel de silice, en éluant par 6,3 litres d'un mélange cyclohexane - acétate d'éthyle (90-10 en volumes) et en recueillant des fractions de 250 $cm^3$. Les fractions comprises entre 2,8 et 6,3 litres sont concentrées à sec.

On obtient ainsi 50,6 g d'un mélange d'isomères E et Z du (dihydro-3,4 2H-benzopyran-1 ylidène-4) acétate d'éthyle sous forme d'une huile jaune pâle.

Spectre de RMN (400 MHz, $CDCl_3$, $\delta$ en ppm) :

Isomère E (75 %) :
6,8 à 7,61 (mt, 4H aromatiques)
6,36 (s, =CH-CO-)
4,23 (mt, -O-CH₂-)
4,23 (mt, -CO-OCH₂-CH₃)
3,41 (mt, -CH₂- en -3)
1,32 (mt, -CO-OCH₂-CH₃)

Isomère Z (25 %) :
6,8 à 7,83 (mt, 4H aromatiques)
5,61 (s, =CH-CO-)
4,38 (t, -O-CH₂-)
4,23 (mt, -CO-OCH₂-CH₃)
2,65 (t, -CH₂- en -3)
1,32 (mt, -CO-OCH₂-CH₃)

EXEMPLE 2

On opère comme à l'exemple 1, mais à partir de 2 g de (bromo-2 éthyl)-4 dihydro-3,4 2H-benzopyranne, de 2 g de N-(pipéridyl-4) acétanilide, de 2 g de carbonate de potassium sec et de 1,38 g d'iodure de potassium dans 50 cm3 de butanone-2.

On reprend l'huile obtenue par 10 cm3 d'une solution (5N) de carbonate de potassium et ajoute 100 cm3 d'eau puis on extrait par deux fois 75 cm3 d'éther éthylique. Les phases organiques réunies sont ensuite séchées sur sulfate de magnésium puis concentrées à sec sous pression réduite (5,2 kPa).

L'huile obtenue est chromatographiée sur une colonne de 2,8 cm de diamètre contenant 25 g de gel de silice en éluant par un mélange dichlorométhane - isopropanol (90-10 en volumes) et en recueillant des fractions de 30 $cm^3$. Les fractions comprises entre 60 et 240 cm3 sont concentrées à sec.

On dissout l'huile obtenue dans le minimum d'acétone à 40 °C et ajoute une solution de 0,71 g d'acide oxalique dans 10 cm3 d'acétone.

On obtient ainsi 3,05 g d'oxalate acide de N-{[(dihydro-3,4 2H-benzopyran-1 yl-4)-2 éthyl]-1 pipéridyl-4} acétanilide sous forme d'un solide blanc fondant à 183 °C.

Le N-(pipéridyl-4) acétanilide peut être préparé selon méthode décrite dnas le brevet français M 2430.

EXEMPLE 3

On chauffe à reflux pendant 18 heures, 1,47 g de (bromo-2 éthyl)-4 dihydro-3,4 2H-benzopyranne, 1,83 g de de chlorhydrate de (diméthoxy-3,4 phényl)-1 (pipéridyl-4)-3 propanone-1, 1,51 g de carbonate de potassium sec et 1 g d'iodure de potassium dans 50 cm3 de butanone-2.

On filtre le mélange réactionnel sur verre fritté puis, après évaporation du solvant sous pression réduite (5,2 kPa), on reprend le mélange réactionnel par 20 cm3 d'eau distillée et extrait par trois fois 70 cm3 d'acétate d'éthyle. La phase organique est ensuite séchée sur sulfate de magnésium puis concentrée à sec sous pression réduite (5,2 kPa).

Le résidu obtenu est purifié par chromatographie sur une colonne de 5,5 cm de diamètre contenant 200 g de gel de silice (32-63 $\mu$) en éluant par 1,6 litres d'un mélange dichlorométhane -éthanol (95-5 en volumes) et ensuite 4,4 litres d'un mélange dichlorométhane - éthanol (90-10 en volumes) et en recueillant des fractions de 200 cm3. Les fractions comprises entre 2,6 et 4,4 litres sont concentrées à sec.

On dissout le produit obtenu dans le minimum d'acétone à 40 °C et ajoute une solution de 0,6 g d'acide oxalique dans l'acétone. Le précipite blanc formé est filtré sur verre fritté puis est ensuite recristallisé une première fois dans 250 cm3 d'éthanol et une deuxième fois dans 100 cm3 d'un mélange

d'éthanol et de butanone-2 (75-25 en volumes).

On obtient ainsi 2,65 g d'oxalate acide de {[(dihydro-3,4 2H-benzopyran-1 yl-4)-2 éthyl]-1 pipéridyl-4}-3 (diméthoxy-3,4 phényl)-1 propanone sous forme d'un solide blanc fondant à 175° C.

La (diméthoxy-3,4 phényl)-1 (pipéridyl-4)-3 propanone-1 peut être préparée de la manière suivante :

A une solution de 140 cm3 de diméthoxy-1,2 benzène dans 200 cm3 de dichlorométhane et de 75 g de chlorure de l'acide benzoyl-1 pipéridyl-4 propionique est ajouté, peu à peu, par fractions de 5-6 g, 47 g de chlorure d'aluminium.

On chauffe ensuite 9 heures à ébullition puis abandonne le mélange réactionnel une nuit à température ambiante. On verse alors le mélange réactionnel dans la glace et après décantation et lavages à l'eau distillée, la phase organique est concentrée à sec.

On reprend l'huile obtenue par 200 cm3 d'une solution 3N de soude et extrait par l'acétate d'éthyle. La phase organique est séparée puis, après lavages à l'eau distillée, elle est concentrée à sec et l'huile obtenue est portée à reflux pendant 48 heures dans 1,2 litres d'une solution 3,25N d'acide chlorhydrique.

Après refroidissement, on filtre les cristaux d'acide benzoïque et alcalinise le filtrat par une solution de soude concentrée. Après trois extractions par le dichlorométhane, la phase organique est lavée à l'eau puis séchée sur sulfate de magnésium. On concentre à sec et l'huile obtenue est reprise par une solution 4N d'acide chlorhydrique dans l'éthanol.

On obtient ainsi 8,49 g de chlorhydrate de la (diméthoxy-3,4 phényl)-1 (pipéridyl-4)-3 propanone sous forme de cristaux blancs fondant à 193° C.

Le chlorure de l'acide benzoyl-1 pipéridyl-4 propionique peut être préparé de la manière suivante :

On chauffe à reflux, pendant 2 heures, 75,4 g d'acide benzoyl-1 pipéridyl-4 propionique avec 84 cm3 de chlorure de thionyle dans 400 cm3 de chloroforme. Après concentration à sec sous pression réduite, on obtient 75 g du chlorure de l'acide benzoyl-1 pipéridyl-4 propionique sous forme d'un solide brun qui est utilisé tel quel pour l'étape suivante.

L'acide benzoyl-1 pipéridyl-4 propionique peut être préparé selon les méthodes décrites dans Beil., 22 - (III/IV), 161 et par C.F. Koelschr, J. Am. Chem. Soc., 65, 2460 (1943).

## EXEMPLE 4

On opère comme à l'exemple 3, mais à partir de 1,60 g de (bromo-2 éthyl)-4 dihydro-3,4 2H-benzopyranne, 2,30 g de chlorhydrate de N-(fluoro-4 phényl) N-pipéridyl-4 fluoro-4 benzamide, 1,80 g de carbonate de potassium sec et 0,80 g d'iodure de potassium dan 50 $cm^3$ de butanone-2.

Après 18 heures de reflux, on filtre le mélange réactionnel sur verre fritté puis après évaporation du solvant sous pression réduite (5,2 kPa) on reprend l'huile jaune obtenue par 100 $cm^3$ de dichlométhane et lave par une solution ammoniacale à 20 %. Après lavage à l'eau, la phase organique est ensuite séchée sur sulfate de magnésium puis concentrée à sec sous pression réduite (5,2 kPa). L'huile jaune obtenue est purifiée par chromatographie sur colonne de 5,5 cm de diamètre contenant 100 g de gel de silice (63-200 $\mu$) en éluant par 810 $cm^3$ d'un mélange dichlorométhane-éthanol (95-5 en volumes) et en recueillant des fractions de 30 $cm^3$. des fractions comprises entre 200 et 810 $cm^3$ sont concentrées à sec.

On reprend l'huile jaune obtenue dans le minimum d'acétone et ajoute 0,90 g d'acide oxalique dissous dans l'acétone. Le précipité blanc formé est filtré sur verre fritté puis est ensuite recristallisé dans 50 $cm^3$ d'éthanol.

On obtient ainsi 2 g d'oxalate acide de N-{[dihydro-3,4 2H-benzopyran-1 yl-4)-2 éthyl]-1 pipéridyl-4} N-(fluoro-4 phényl) fluoro-4 benzamide sous forme d'une poudre blanche fondant à 150° C.

Le chlorhydrate de N-(fluoro-4 phényl) N-pipéridyl-4 fluoro-4 benzamide peut être préparé de la manière suivante :

3,2 g de chlorhydrate de N-(benzyl-1 pipéridyl-4) N-(fluoro-4 phényl) fluoro-4 benzamide dans 50 $cm^3$ d'éthanol à 95 % sont hydrogénés à 60° C, sous pression atmosphérique en présence de 0,5 g d'hydroxyde de palladium. Après filtration sur Kieselguhr et concentration à sec sous pression réduite (5,2 kPa) on obtient 2,3 g de chlorhydrate de N-(fluoro-4 phényl) N-(pipéridyl-4) fluoro-4 benzamide sous forme d'une poudre blanche fondant à 170° C.

Le chlorhydrate de N-(benzyl-1 pipéridyl-4) N-(fluoro-4 phényl) fluoro-4 benzamide peut être préparé de la manière suivante :

2,8 g de N-benzyl (fluoro-4 anilino)-4 pipéridine dissous dans 30 $cm^3$ de trichlorométhane sont agités pendant 18 heures avec 2 $cm^3$ du chlorure de l'acide fluoro-4 benzoïque et en présence de 5 $cm^3$ de triéthylamine. Le mélange réactionnel est ensuite lavé à deux reprises par 30 $cm^3$ d'une solution (3N) de soude puis par 30 $cm^3$ d'eau. La phase organique est alors séchée sur sulfate de magnésium puis

concentrée sous pression résuite (5,2 kPa). Par addition de 5 $cm^3$ d'une solution 3,4 N d'acide chlorhydrique dans l'isopropanol et recristallisation dans 100 $cm^3$ d'un mélange acétone-éthanol (50-50 en volumes), on obtient 3,2 g de chlorhydrate de N-(benzyl-1 pipéridyl-4) N-(fluoro-4 phényl) fluoro-4 benzamide sous forme d'une poudre blanche fondant à 210 °C.

La N-benzyl (fluoro-4 anilino)-4 pipéridine peut être préparés de la manière suivante :

2 g de benzylpipéridone en solution dans 10 $cm^3$ de toluène sont agités sous argon pendant 48 heures à température ambiante en présence de 1,4 g de fluoro-4 aniline et de 4 g de tamis moléculaire 5 Å. Après filtration et concentration à sec sous pression réduite (5,2 kPa) on obtient des cristaux jaunes qui sont alors dissous dans 30 $cm^3$ de méthanol. Cette solution est ensuite ajoutée à une solution refroidie à 0-5 °C, de 1,34 g de cyanoborohydrure de sodium et de 1,4 g de chlorure de zinc dans 20 $cm^3$ de méthanol. Après 20 heures à température ambiante, on reprend par 5 $cm^3$ d'une solution 10N de soude et par 20 $cm^3$ d'eau et chauffe à reflux pendant 1 heure. On filtre sur fritté et le filtrat est extrait à deux reprises par 100 $cm^3$ de dichlorométhane. La phase organique est ensuite séchée sur sulfate de magnésium puis concentrée à sec sous pression réduite (5,2 kPa). On obtient ainsi 2,8 g de N-benzyl (fluoro-4 anilino)-4 pipéridine sous forme d'une poudre blanchâtre fondant à 84 °C.

## EXEMPLE 5

On opère comme à l'exemple 4, mais à partir de 1,6 g de (bromo-2 éthyl)-4 dihydro-3,4 2H-benzopyranne, 2,21 g de chlorhydrate de N-phényl N-(pipéridyl-4) fluoro-4 benzamide, 1,8 g de carbonate de potassium sec et 0,8 g d'iodure de potassium dans 50 $cm^3$ de butanone-2. On reprend l'huile obtenue dans le minimum d'acétone et ajoute 0,6 g d'acide oxalique dissous dans l'acétone. Le précipité blanc formé est filtré sur verre fritté puis recristallisé dans 50 $cm^3$ d'éthanol. On obtient ainsi 2,7 g d'oxalate acide de N-{[dihydro-3,4 2H-benzopyran-1 yl-4)-2 éthyl]-1 pipéridyl-4} N-phényl fluoro-4 benzamide sous forme d'un solide blanc fondant à 161 °C.

Le chlorhydrate de N-phényl N-(pipéridyl) fluoro-4 benzamide est préparé comme décrit à l'exemple 4 pour le N-(fluoro-4 phényl) N-(pipéridyl-4) fluoro-4 benzamide.

## EXEMPLE 6

On opère comme à l'exemple 4, mais à partir de 1,6 g de (bromo-2 éthyl)-4 dihydro-3,4 2H-benzopyranne, 2,1 g de chlorhydrate de N-phényl N-(pipéridyl-4) benzamide, 1,8 g de carbonate de potassium sec et 0,8 g d'iodure de potassium dans 50 $cm^3$ de butanone-2. On reprend l'huile obtenue dans le minimum d'acétone et ajoute 0,6 g d'acide oxalique dissous dans l'acétone. Le précipité blanc formé est filtré sur verre fritté puis recristallisé dans 45 $cm^3$ d'éthanol.

On obtient ainsi 2,7 g d'oxalate acide de N-{[dihydro-3,4 2H-benzopyran-1 yl-4)-2 éthyl]-1 pipéridyl-4} N-phényl benzamide sous forme d'un solide blanc fondant à 135 °C.

Le chlorhydrate de N-phényl N-(pipéridyl-4) benzamide est préparé comme décrit à l'exemple 4 pour le N-(fluoro-4 phényl) N-(pipéridyl-4) fluoro-4 benzamide.

## EXEMPLE 7

On opère comme à l'exemple 1, mais à partir de 2,5 g de (bromo-2 éthyl)-4 dihydro-3,4 2H-benzopyranne, de 3,26 g de N-(diméthyl-2,6 phényl) N-(pipéridyl-4) fluoro-4 benzamide, de 2,76 g de carbonate de potassium sec et de 1,66 g d'iodure de potassium dans 25 $cm^3$ de butanone-2 en chauffant pendant 3 heures.

La butanone-2 est évaporée sous pression réduite (5,2 kPa) le résidu est repris dans 50 $cm^3$ d'eau et extrait avec 100 $cm^3$ puis deux fois 50 $cm^3$ de dichlorométhane. Les phases organiques réunies sont séchées sur sulfate de magnésium puis concentrées à sec sous pression réduite (5,2 kPa). L'huile obtenue est chromatographiée sur une colonne de 4 cm de diamètre contenant 150 g de gel de silice, en éluant par un mélange dichlorométhane-éthanol (98-2 en volumes) et en recueillant des fractions de 25 $cm^3$. Les fractions comprises entre 125 et 1100 $cm^3$ sont concentrées à sec. L'huile obtenue est dissoute dans 45 $cm^3$ d'éthanol absolu et additionné de 0,83 g d'acide oxalique à chaud. On laisse cristalliser à +5 °C.

On obtient ainsi 3,75 g d'oxalate acide de N-{[dihydro-3,4 2H-benzopyran-1 yl-4)-2 éthyl]-1 pipéridyl-4} N-(diméthyl-2,6 phényl) fluoro-4 benzamide, sous forme d'un solide blanc fondant à 205 °C.

Le N-(diméthyl-2,6 phényl) N-pipéridyl-4 fluoro-4 benzamide peut être préparé de la manière suivante :

Une solution de 8,57 g de N-(benzyl-1 pipéridyl-4) N-(diméthyl-2,6 phényl) fluoro-4 benzamide dans 100 $cm^3$ d'éthanol est additionnée de 2 $cm^3$ d'une solution aqueuse d'acide chlorhydrique 12N et de 1 g de palladium sur charbon à 10 %. Cette suspension est soumise à l'action de l'hydrogène sous pression atmosphérique à 55°C. Après 4 heures, le volume théorique a été absorbé. On refroidit et filtre le catalyseur qui est lavé trois fois avec 20 $cm^3$ d'éthanol.

L'éthanol est évaporé sous pression réduite (5,2 kPa). Le solide obtenu est repris dans 50 $cm^3$ d'eau, additionné de 2,5 $cm^3$ de soude 10N et extrait 7 fois avec 50 $cm^3$ de dichlorométhane. Les phases organiques jointes sont concentrées à sec sous pression réduite (5,2 kPa).

On obtient 6,6 g de N-(diméthyl-2,6 phényl) N-(pipéridyl-4) fluoro-4 benzamide sous la forme d'une huile brune.

Spectre de RMN (300 MHz, $CDCl_3$, δ en ppm, J en Hz)

- 6,65 à 7,3 (Mt, 7H aromatiques)
- 4,1 (Mt, 1H, >N-CH< de la pipéridine)
- 3,06 (D large, J = 12,5, 2H équatoriaux des >N-$CH_2$- de la pipéridine)
- 2,65 (T large, J = 12,5 + S, 3H axiaux des >N-$CH_2$- de la pipéridine + -NH-)
- 2,17 (S, 6H : Ar-$CH_3$)
- 1,88 (D large, J = 12,5, 2H : H équatoriaux des -$CH_2$- de la pipéridine)
- 1,66 (Mt, 2H, H axiaux des -$CH_2$- de la pipéridine).

Le N-(diméthyl-2,6 phényl) N-(benzyl-1 pipéridyl-4) fluoro-4 benzamide peut être préparé de la manière suivante :

A une solution de 6,5g de N-(diméthyl-2,6 phényl) (benzyl-1 pipéridyl-4) amine dans 100 $cm^3$ de trichlorométhane, on additionne 4,5 $cm^3$ de triéthylamine puis 3,1 $cm^3$ de chlorure de l'acide fluoro-4 benzoïque, en maintenant à +25°C. Après 30 minutes, on verse 100 $cm^3$ d'eau, puis après 16 heures, on décante et réextrait la phase aqueuse deux fois avec 50 $cm^3$ de trichlorométhane. Les phases organiques réunies sont séchées sur sulfate de magnésium puis concentrées à sec sous pression réduite (5,2 kPa).

L'huile obtenue est chromatographiée sur une colonne de 6 cm de diamètre contenant 500 g de gel de silice, en éluant d'abord avec 2750 $cm^3$ du mélange dichlorométhane-éthanol (98-2 en volumes) puis du même mélange de solvants (95-5 en volumes). On recueille des fractions de 125 $cm^3$. Les fractions comprises entre 3500 et 4500 $cm^3$ sont concentrées à sec. On obtient 8,7 g de N-(benzyl-1 pipéridyl-4) N-(diméthyl-2,6 phényl) fluoro-4 benzamide sous la forme d'une laque brune.

Spectre de RMN (300 MHz, DMSO d6, δ en ppm, J en Hz)

- 6,95 à 7,4 (Mt, 12H aromatiques)
- 3,9 (Mt, 1H : >CH-N< de la pipéridine)
- 3,47 (S, 2H : >N-$CH_2$- exo)
- 2,86 (D large, J = 12,5, 2H, H équatoriaux des >N-$CH_2$- de la pipéridine)
- 2,23 (S, 6H, Ar-$CH_3$)
- 2,03 (DT, J = 12,5 et 3, 2H : H axiaux des >N-$CH_2$- de la pipéridine)
- 1,7 à 1,9 (Mt, 4H, -$CH_2$- de la pipéridine).

La N-(diméthyl-2,6 phényl) N-(benzyl-1 pipéridyl-4) amine peut être préparée de la manière suivante :

A une solution de 18,9 g de N-benzylpipéridone-4 dans 100 $cm^3$ de toluène, on additionne 14,8 $cm^3$ de diméthyl-2,6 aniline pendant 2 heures en recueillant l'eau formée à l'aide d'un "Dean-Stark". On évapore le toluène sous pression réduite (5,2 kPa).

L'huile obtenue est dissoute dans 100 $cm^3$ de méthanol et additionnée à un mélange de 12,65 g de cyanoborohydrure de sodium et de 13,6 g de chlorure de zinc dans 150 $cm^3$ de méthanol, à une température inférieure à 20°C. Après 2 heures à 20°C, on verse 50 $cm^3$ d'une solution de soude 10N, additionne 100 $cm^3$ d'eau et chauffe à reflux pendant 30 minutes. On refroidit à température ambiante pendant 16 heures et additionne 200 $cm^3$ de dichlorométhane. La phase aqueuse est décantée et réextraite deux fois avec 100 $cm^3$ de dichlorométhane. Les phases organqiues sont réunies et séchées sur sulfate de magnésium puis concentrées à sec (5,2 kPa). L'huile obtenue est chromatographiée sur une colonne de 7 cm de diamètre contenant 900 g de gel de silice en éluant d'abord avec 2500 $cm^3$ de dichlorométhane pur puis du mélange dichlorométhane-éthanol (97-3 en volumes), et en recueillant des fractions de 250 $cm^3$. Les fractions comprises entre 7250 $cm^3$ et 10500 $cm^3$ réunies sont concentrées à sec pour donner 6,5 g de N-(diméthyl-2,6 phényl) N-(benzyl-1 pipéridyl-4) amine, sous forme d'une huile brune.

Spectre de RMN (250 MHz, $CDCl_3$, δ ppm et J en Hz)

- 6,75 à 7,45 (Mt, 8H aromatiques)
- 3,54 (S, 2H, >N-$CH_2$- exo)
- 3,02 (Mt, 1H, >N-CH< de la pipéridine)

- 2,92 (D large, J = 12,5, 2H, H équatoriaux des ≻N-$CH_2$- de la pipéridine)
- 2,77 (Mf, 1H, -NH-)
- 2,3 (S, 6H, Ar-$CH_3$)
- 2,03 (DT, J = 12,5 et 2, 2H : H axiaux des ≻N-$CH_2$- de la pipéridine)
- 1,93 (D large, J = 12,5, 2H : H équatoriaux des -$CH_2$- de la pipéridine)
- 1,5 (Mt, 2H : H axiaux des -$CH_2$- de la pipéridine).

EXEMPLE 8

On opère comme à l'exemple 4, mais à partir de 1,6 g de (bromo-2 éthyl)-4 dihydro-3,4 2H-benzopyranne, 2,95 g de chlorhydrate de N-(chloro-4 phényl) N-(pipéridyl-4) méthanesulfonamido-4 benzamide, 0,9 g de carbonate de potassium sec et 0,8 g d'iodure de potassium dans 50 $cm^3$ de butanone-2. Le résidu obtenu est repris par 100 $cm^3$ d'éthanol et 6,6 $cm^3$ d'une solution 1N d'acide chlorhydrique puis concentré jusqu'au début de la cristallisation. Le précipité blanc formé est filtré sur verre fritté puis est ensuite recristallisé sans 100 $cm^3$ de méthanol.

On obtient ainsi 2,1 g de chlorhydrate de N-{[(dihydro-3,4 2H-benzopyran-1 yl-4)-2 éthyl]-1 pipéridyl-4} N-(chloro-4 phényl) méthanesulfonamido-4 benzamide fondant aux environs de 260° C.

EXEMPLE 9

On opère comme à l'exemple 4, mais à partir de 1,6 g de (bromo-2 éthyl)-4 dihydro-3,4 2H-benzopyranne, 2,42 g de chlorhydrate de N-(chloro-4 phényl) N-pipéridyl-4 phénylacétamide, 1,80 g de carbonate de potassium sec et 0,80 g d'iodure de potassium dans 50 $cm^3$ de butanone-2.

On reprend l'huile obtenue dans le minimum d'acétone et ajoute 0,6 g d'acide oxalique dissous dans l'acétone. Le précipité blanc formé est filtré sur verre fritté puis recristallisé dans 50 $cm^3$ d'éthanol.

On obtient ainsi 2,3 g d'oxalate acide de N-{[dihydro-3,4 2H-benzopyran-1 yl-4)-2 éthyl]-1 pipéridyl-4} N-(chloro-4 phényl) phénylacétamide sous forme d'un solide blanc fondant à 185° C.

le chlorhydrate de N-(chloro-4 phényl) N-(pipéridyl-4) phénylacétamide a été préparé par analogie avec le N-(chloro-3 phényl) N-(pipéridyl-4) phénylacétamide Chem. Abstr., 93 132380.

EXEMPLE 10

On opère comme à l'exemple 1, mais à partir de 1,94 g de (bromo-2 éthyl)-4 dihydro-3,4 2H-benzopyranne, de 2,21 g de chlorhydrate de N-isopropyl N-(pipéridyl-4) fluoro-4 benzamide, de 3,05 g de carbonate de potassium sec et de 1,22 g d'iodure de potassium dans 20 $cm^3$ de butanone-2, en agitant pendant 4 heures 30 minutes. On évapore la butanone sous pression réduite (5,2 kPa), et reprend le résidu dans 50 $cm^3$ d'eau que l'on extrait avec 50 $cm^3$ puis deux fois 25 $cm^3$ de dichlorométhane. Les phases organiques réunies sont séchées sur sulfate de magnésium sec puis concentrées à sec sous pression réduite (5,2 kPa). L'huile obtenue est chromatographiée sur une colonne de 3 cm de diamètre contenant 100 g de gel de silice, en éluant avec un mélange dichlorométhane-éthanol (95-5 en volumes) et en recueillant des fractions de 25 $cm^3$. Les fractions comprises entre 150 et 950 $cm^3$ sont concentrées à sec. L'huile obtenue est dissoute dans 30 $cm^3$ d'acétate d'éthyle et additionnée de 1,5 $cm^3$ d'une solution d'acide chlorhydrique 5N dans l'isopropanol.

On obtient ainsi 3 g de chlorhydrate de N-{[(dihydro-3,4 2H-benzopyran-1 yl-4)-2 éthyl]-1 pipéridyl-4} N-isopropyl fluoro-4 benzamide, sous la forme d'un solide blanc fondant à 165° C.

Le chlorhydrate de N-isopropyl, N-(pipéridyl-4) fluoro-4 benzamide peut être préparé de la manière suivante :

A une solution de 3,35 g de N-(benzyl-1 pipéridyl-4) N-isopropyl fluoro-4 benzamide dans 100 $cm^3$ d'éthanol absolu, on additionne 20 $cm^3$ d'acide chlorhydrique aqueux 3N puis 1,5 g de palladium sur charbon à 10 %. Cette suspension est soumise à l'action de l'hydrogène, sous pression atmosphérique à une température de 55° C. Après 4 heures, le volume théorique a été absorbé. On refroidit et filtre le catalyseur qui est lavé à l'éthanol puis à l'eau. On évapore l'éthanol sous pression réduite (5,2 kPa). Le solide obtenu est recristallisé dans 20 $cm^3$ d'éthanol absolu.

On obtient ainsi 2,6 g de chlorhydrate de N-isopropyl N-(pipéridyl-4) fluoro-4 benzamide sous forme d'un solide blanc fondant au-delà de 260° C.

Spectre de RMN (200 MHz, DMSO d6, δ en ppm et J en Hz) :
- 1,2 (Mf, 6H, $-CH_3$ de l'isopropyle)
-1,7 (D large, J = 16, 2H, - H équatoriaux des $-CH_2-$ de la pipéridine)
-2,65 à 3,20 (Mt, 4H, H axiaux des $-CH_2-$ et des $>N-CH_2-$ de la pipéridine)
- 3,28 (D large, J = 16, H équatoriaux des $>N-CH_2-$ de la pipéridine)
-3,4 à 3,8 (Mt, 2H, $>CH-N<$ de la pipéridine et - $>H$-isopropyle)
-7,2 à 7,5 (Mt, aromatiques)

Le N-(benzyl-1 pipéridyl-4) N-isopropyl fluoro-4 benzamide peut être préparé de la manière suivante :

A une solution de 3,83 g de dichlorhydrate hydraté de benzyl-1 isopropylamino-4 pipéridine et de 15 $cm^3$ de triéthylamine dans 50 $cm^3$ de trichlorométhane, on additionne 3,7 $cm^3$ de chlorure de l'acide fluoro-4 benzoïque. Après 6 heures d'agitation à 20° C, on verse 50 $cm^3$ d'eau et laisse reposer pendant 16 heures. On décante et extrait la phase aqueuse avec 50 $cm^3$ de trichlorométhane. Les phases organiques sont lavées avec 50 $cm^3$ d'eau puis séchées sur sulfate de magnésium, et concentrées à sec sous pression réduite (5,2 kPa). L'huile obtenue est chromatographiée sur une colonne de 4 cm de diamètre contenant 200 g de gel de silice, en éluant d'abord avec 1400 $cm^3$ d'un mélange dichlorométhane-éthanol (95-5 en volumes) et en recueillant des fractions de 60 $cm^3$. Les fractions comprises entre 480 et 1980 $cm^3$ sont concentrées à sec pour donner un solide ocre que l'on recristallise dans un mélange éther diisopropylique-éthanol (95-5 en volumes) pour obtenir 1,2 g de N-(benzyl-1 pipéridyl-4) N-isopropyl fluoro-4 benzamide, sous la forme d'un solide blanc fondant à 98° C.

Le dichlorhydrate de benzyl-1 isopropylamino-4 pipéridine peut être préparé de la façon suivante :

A une solution de 6,8 g de chlorhydrate de N-benzyl pipéridone-4 dans 100 $cm^3$ d'éthanol, on additionne 14,3g de chlorhydrate d'isopropylamine et refroidit à +10° C. On additionne alors 1,9 g de cyanoborohydrure de sodium puis agite 16 heures à 20° C.

On verse 50 $cm^3$ de soude 10N et agite pendant 22 heures, à 20° C. On évapore l'éthanol sous pression réduite (5,2 kPa) et extrait l'huile résiduelle trois fois avec 50 $cm^3$ de dichlorométhane. Les phases organiques réunies sont séchées sur sulfate de magnésium et concentrées à sec. L'huile résiduelle est dissoute dans 50 $cm^3$ d'éthanol absolu et additionnée de 11,5 $cm^3$ d'une solution 5N d'acide chlorhydrique dans l'isopropanol. On obtient ainsi 4,24 g de dichlorhydrate hydraté de benzyl-1 isopropylamino-4 pipéridine sous la forme d'un solide blanc fondant au-dessus de 260° C.

Spectre de RMN (200MHz, DMSO d6, δ en ppm et J en Hz)
(A température ordinaire, on observe un mélange de deux conformères dans les proportions 85/15)
- 7,30 à 7,80 (Mt : 5H aromatiques)
- 4,26 et 4,46 (D et AB limite : 2H en totalité et dans un rapport 85/15 respectivement ; $>N-CH_2-$ exo)
- 2,80 à 3,50 (Mt, 6H : $>N-CH_2-$ et $>N-CH<$ de la pipéridine et $>CH-$ de l'isopropyle)
- 1,90 à 2,30 (Mt, 4H : $-CH_2-$ de la pipéridine)
- 1,28 et 1,32 (2D, J = 7, 6H en totalité et dans un rapport 85/15 respectivement, $-CH_3$ de l'isopropyle).

## EXEMPLE 11

On opère comme à l'exemple 4, mais à partir de 1,6 g de (bromo-2 éthyl)-4 dihydro-3,4 2H-benzopyranne, 2,4 g de chlorhydrate de phényl-1 (pipéridyl-4)-1 (diméthyl-2,6 phényl)-3 urée, 1,8 g de carbonate de potassium sec et 0,8 g d'iodure de potassium dans 50 $cm^3$ de butanone-2.

On reprend le résidu solide obtenu dans le minimum d'acétone et ajoute 0,6 g d'acide oxalique dissous dans l'acétone.

Le précipité blanc formé est filtré sur verre fritté puis est ensuite recristallisé dans 150 $cm^3$ d'éthanol aqueux à 95 %.

On obtient ainsi 2,1 g d'oxalate acide de {[dihydro-3,4 2H-benzopyran-1 yl-4)-2 éthyl]-1 pipéridyl-4}-1 phényl-1 (diméthyl-2,6 phényl)-3 urée sous forme d'une poudre blanche fondant avec décomposition à 190° C.

Le chlorhydrate de phényl-1 (pipéridyl-4)-1 (diméthyl-2,6 phényl)-3 urée est préparé par analogie avec la méthode décrite dans Chem. Abstr. 88, 022640.

## EXEMPLE 12

On opère comme à l'exemple 1, mais à partir de 2,65 g de (bromo-2 éthyl)-4 dihydro-3,4 2H-benzopyranne, de 2,46 g de N-(diméthyl-2,6 phényl) pipéridine-4 acétamide, de 2,76 g de carbonate de

potassium sec, de 1,66 g d'iodure de potassium dans 25 $cm^3$ de diméthylformamide sec, en chauf fant pendant 4 heures à 60° C. On évapore le diméthylformamide sous pression réduite (1 kPa) et reprend le résidu dans 50 $cm^3$ d'eau que l'on extrait trois fois avec 50 $cm^3$ de dichlorométhane. Les phases organiques réunies sont séchées sur sulfate de magnésium puis concentrées à sec sous pression réduite (5 kPa). Le solide obtenu est chromatographié sur une colonne de 4 cm de diamètre, contenant 120 g de gel de silice, en éluant par un mélange toluène-diéthylamine (95-5 en volumes) et en recueillant des fractions de 25 $cm^3$. Les fractions comprises entre 175 et 1000 $cm^3$ sont concentrées à sec. le solide obtenu est recristallisé dans 20 $cm^3$ d'acétone.

On obtient ainsi 2,55 g de [(dihydro-3,4 2H-benzopyran-1 yl-4)-2 éthyl]-1 N-(diméthyl-2,6 phényl) pipéridine-4 acétamide, sous la forme d'un solide blanc fondant à 136° C.

Le N-(diméthyl-2,6 phényl) pipéridine-4 actamide est préparé comme décrit dans Chem. Abstr., 76, 152718x.

## EXEMPLE 13

On opère comme à l'exemple 1, mais à partir de 2,41 g de (bromo-2 éthyl)-4 dihydro-3,4 2H-benzopyranne, de 2,64 g de dichlorhydrate de N-(nicotinoyl) pipérazine, de 4,15 g de carbonate de potassium sec et de 1,66 g d'iodure de potassium dans 25 $cm^3$ de butanone-2, en chauffant pendant 3 heures 30 minutes.

On évapore la butanone sous pression réduite (5,2 kPa) et reprend l'huile obtenue dans 50 $cm^3$ d'eau que l'on extrait avec 50 $cm^3$ puis deux fois 25 $cm^3$ de dichlorométhane. Les phases organiques réunies sont séchées sur sulfate de magnésium puis concentrées à sec sous pression réduite (5,2 kPa).

L'huile obtenue est chromatographiée sur une colonne de 4 cm de diamètre contenant 200 g de gel de silice, en éluant par un mélange toluène-diéthylamine (95-5 en volumes) et en recueillant des fractions de 60 $cm^3$. les fractions comprises entre 1140 et 1500 $cm^3$ sont concentrées à sec.

L'huile obtenue est dissoute dans 20 $cm^3$ d'éthanol absolu et additionnée de 2,5 $cm^3$ d'une solution 5N d'acide chlorhydrique dans l'isopropanol.

On obtient ainsi 2,35 g de dichlorhydrate de [(dihydro-3,4 2H-benzopyran-1 yl-4)-2 éthyl]-1 nicotinoyl-4 pipérazine monohydratée, sous la forme de cristaux blancs fondant à 190° C en se décomposant.

La N-(nicotinoyl) pipérazine est préparée comme décrit dans Chem. Abstr., 42, 6002g (1948).

## EXEMPLE 14

On opère comme à l'exemple 1, mais à partir de 2,9 g de (bromo-2 éthyl)-4 dihydro-3,4 2H-benzopyranne, de 2,05 g de benzoyl-4 pipéridine, de 3 g de carbonate de potassium sec, de 1,8 g d'iodure de potassium, dans 25 $cm^3$ de butanone-2, en chauffant pendant 1 heure 30 minutes. On évapore la butanone sous pression réduite (5,2 kPa) et reprend l'huile obtenue dans 50 $cm^3$ d'eau que l'on extrait avec 50 $cm^3$ puis deux fois 25 $cm^3$ de dichlorométhane. Les phases organiques réunies sont séchées sur sulfate de magnésium puis concentrées à sec sous pression réduite (5,2 XPa). L'huile obtenue est chromatographiée sur une colonne de 4 cm de diamètre contenant 200 g de gel de silice, en éluant avec un mélange dichlorométhane-éthanol (95-5 en volumes), et en recueillant des fractions de 60 $cm^3$. Les fractions comprises entre 360 et 1560 $cm^3$ sont concentrées à sec (5,2 kPa). L'huile obtenue est dissoute dans 40 $cm^3$ d'acétone et additionnée de 2,2 $cm^3$ d'une solution 5N d'acide chlorhydrique dans l'isopropanol.

On obtient ainsi 2,65 g de chlorhydrate de benzoyl-4 [(dihydro-3,4 2H-benzopyran-1 yl-4)-2 éthyl]-1 pipéridine, sous forme d'un solide blanc fondant à 210° C en se décomposant.

## EXEMPLE 15

On opère comme à l'exemple 1, mais à partir de 2,41 g de (bromo-2 éthyl)-4 dihydro-3,4 2H-benzopyranne, de 3,8 g de paratoluènesulfonate de (fluoro-4 benzoyl)-4 pipéridine, de 2,75 g de carbonate de potassium sec, de 1,66 g d'iodure de potassium, dans 25 $cm^3$ de butanone-2, en chauffant pendant 2 heures. On évapore la butanone sous pression réduite (5,2 kPa) et reprend l'huile obtenue dans 50 $cm^3$ d'eau que l'on extrait avec 50 $cm^3$ puis deux fois 25 $cm^3$ de dichlorométhane. les phases organiques réunies sont séchées sur sul fate de magnésium puis concentrées à sec sous pression réduite (5,2 kPa). Le solide obtenu est dissous dans 70 $cm^3$ d'acétone tiède et additionné de 2,1 $cm^3$ d'une solution 5N d'acide

chlorhydrique dans l'isopropanol.

On obtient ainsi 3 g de chlorhydrate de [(dihydro-3,4 2H-benzopyran-1 yl-4)-2 éthyl]-1 (fluoro-4 benzoyl)-4 pipéridine sous le forme d'un solide blanc fondant à 220 °C en se décomposant.

EXEMPLE 16

On opère comme à l'exemple 4, mais à partir de 1,5 g de (bromo-2 éthyl)-4 dihydro-3,4 2H-benzopyranne, 2,16 g d'oxalate acide de (pipéridyl-4) carbonyl-3 indole, 1,71 g de carbonate de potassium sec et 1,085 g d'iodure de potassium dans 50 $cm^3$ de butanone. Le résidu obtenu est repris à chaud par 20 $cm^3$ d'éthanol et 6,2 $cm^3$ d'une solution 1N d'acide chlorhydrique puis concentré jusqu'au début de cristallisation. Le précipité formé est filtré sur verre fritté puis est ensuite recristallisé dans 50 $cm^3$ d'éthanol à 95 %. On obtient ainsi 0,68 g de chlorhydrate de N-{[(dihydro-3,4 2H-benzopyran-1 yl-4)-2 éthyl]-1 pipéridyl-4} carbonyl-3 indole sous forme d'une poudre blanche fondant à 240 °C. Le (pipéridyl-4)carbonyl-3 indole est préparé selon la technique décrite dans Chem. Abstr., 64, 14161f.

EXEMPLE 17

A une solution de 0,63 g de chlorhydrate de benzoyl-4 [(dihydro-3,4 2H-benpyran-1 yl-4)-2 éthyl]-1 pipéridine et de 3,3 $cm^3$ d'une solution de soude N dans 20 $cm^3$ d'éthnaol absolu, on additionne 0,3 g de chlorhydrate d'hydroxylamine et agite à +20 °C pendant 16 heures. Les solvants sont évaporés sous pression réduite (5,2 kPa) et le résidu est repris dans 25 $cm^3$ d'eau que l'on extrait trois fois avec 25 $cm^3$ de dichlorométhane. Les phases organiques réunies sont séchées sur sulfate de magnésium puis concentrées à sec sous pression réduite. Le solide obtenu est dissous dans 10 $cm^3$ d'acétone bouillante et additionné de 0,12 g d'acide oxalique que l'on dissout à ébullition.

On obtient ainsi 0,37 g d'oxalate acide de {[(dihydro-3,4 2H-benzopyran-1 yl-4)-2 éthyl]-1 pipéridyl-4} phényl méthanoxine (Z + E) sous forme d'un solide blanc fondant à partir de 130 °C.

EXEMPLE 18

A une solution refroidie entre 0 et 5 °C de 1,64 g de [(dihydro-3,4 2H-benzopyran-1 yl-4)-2 éthyl]-1 pipérazine dans 25 cm3 de dichlorométhane, on ajoute 1,03 cm3 de triéthylamine puis on introduit, goutte à goutte, 0,85 cm3 de chlorure de l'acide benzènesulfonique.

Après 2 heures à 20 °C, on dilue par 10 cm3 d'eau distillée et ajoute une quantité identique d'une solution 1N de soude puis extrait à deux reprises par 10 cm3 de dichlorométhane. On sèche la phase organique sur sulfate de magnésium puis on concentre à sec sous pression réduite (5,2 kPa).

Le résidu obtenu est chromatographié sur une colonne de 3 cm de diamètre contenant 60 g de gel de silice en éluant par un mélange dichlorométhane - acétone (70-30 en volumes) et en recueillant des fractions de 30 cm3. Les fractions comprises entre 90 et 210 cm3 sont concentrées à sec sous pression réduite (5,2 kPa).

On reprend l'huile obtenue par 20 cm3 d'éthanol et ajoute 3 cm3 d'une solution 5N d'acide chlorhydrique dans l'isopropanol, concentre à sec et recristallise dans 40 cm3 de butanone-2. On obtient ainsi 1,45 g de chlorhydrate de [(dihydro-3,4 2H-benzopyran-1 yl-4)-2 éthyl]-1 (phénylsulfonyl)-4 pipérazine sous forme d'un solide blanc fondant à 164 °C.

La [(dihydro-3,4 2H-benzopyran-1 yl-4)-2 éthyl]-1 pipérazine peut être préparée de la manière suivante :

On opère comme à l'exemple 1 à partir de 9,7 g de (bromo-2 éthyl)-4 dihydro-3,4 2H-benzopyranne, de 10,4 g de pipérazine puis de 13,4 g d'iodure de potassium dans 300 cm3 de butanone-2 mais sans adjonction de carbonate de potassium. L'huile obtenue est chromatographiée sur une colonne de 4,4 cm de diamètre contenant 100 g de gel de silice en utilisant comme éluant un mélange dichlorométhane -éthanol - diéthylamine (80-18-2 en volumes). Les fractions comprises entre 200 et 500 cm3 sont concentrées à sec.

On obtient ainsi 7,1 g de [dihydro-3,4 2H-benzopyran-1 yl-4)-2 éthyl]-1 pipérazine sous forme d'une huile qui est utilisée telle quelle pour l'étape suivante.

EXEMPLE 19

On chauffe à reflux, pendant 12 heures, 2,85 g de [(dihydro-3,4 2H-benzopyran-1 yl-4)-2 éthyl]-1 pipérazine, 2 g de N-(diméthyl-2,6 phényl) chloro-3 propionamide, 1,23 g de carbonate de potassium sec et 1,48 g d'iodure de potassium dans 70 cm3 de butanone-2.

On filtre le mélange réactionnel sur verre fritté puis on évapore le solvant sous pression réduite (5,2 kPa). On reprend l'huile obtenue par 15 cm3 d'une solution 1N de soude puis extrait à deux reprises par 150 cm3 de dichlorométhane. La phase organique est ensuite lavée à l'eau puis séchée sur sulfate de magnésium.

Après évaporation, on obtient une huile qui est purifiée par chromatographie sur une colonne de 4,4 cm de diamètre contenant 100 g de gel de silice, en éluant par un mélange dichlorométhane -isopropanol (80-20 en volumes) et en recueillant des fractions de 50 cm3. Les fractions comprises entre 1,3 et 2 litres sont concentrées à sec.

Après recristallisation dans l'acétate d'isopropyle, on obtient 1 g de {[(dihydro-3,4 2H-benzopyran-1 yl-4)-2 éthyl]-1 pipérazinyl-4} N-(diméthyl-2,6 phényl) propanamide sous forme d'un solide blanc fondant à 130 °C.

La N-(diméthyl-2,6 phényl) chloro-3 propanamide peut être préparée selon la méthode décrite dans Beil, 12 III, 2464.

## EXEMPLE 20

On opère comme à l'exemple 19, mais à partir de 2 g de [(dihydro-3,4 2H-benzopyran-1 yl-4)-2 éthyl]-1 pipérazine, 1,6 g de N-(diméthyl-2,6 phényl) chloroacétamide, 1,12 g de carbonate de potassium sec et 1,34 g d'iodure de potassium dans 80 cm3 de butanone-2.

Après 2 heures 30 minutes de reflux, on obtient un résidu qui est chromatographié sur une colonne de 4,4 cm de diamètre contenant 100 g de gel de silice et en utilisant un mélange acétate d'éthyle - éthanol (90-10 en volumes) comme éluant et en recueillant des fractions de 25 cm3. Les fractions comprises entre 475 et 1000 cm3 sont concentrées à sec sous pression réduite (5,2 kPa).

On reprend l'huile obtenue par 30 cm3 d'éthanol et ajoute 1,3 cm3 d'une solution 5,5N d'acide chlorhydrique dans l'isopropanol.

Après cristallisation, on obtient 1 g de chlorhydrate de {[(dihydro-3,4 2H-benzopyran-1 yl-4)-2 éthyl]-1 pipérazinyl-4} N-(diméthyl-2,6 phényl) acétamide sous forme d'un solide blanc fondant aux environs de 230 °C.

La N-(diméthyl-2,6 phényl) chloroacétamide peut être préparée selon la méthode décrite dans Beil., 12 III, 2464.

## EXEMPLE 21

On opère comme à l'exemple 4, mais à partir de 1,9 g de (bromo-2 éthyl)-4 dihydro-3,4 2H-benzopyranne, de 2 g de chlorhydrate de N-(pipéridyl-4) fluoro-4 benzamide, 2,15 g de carbonate de potassium sec et 0,95 g d'iodure de potassium dans 60 $cm^3$ de butanone-2. L'huile jaune obtenue est purifiée par chromatographie sur colonne de 4 cm de diamètre contenant 110 g de gel de silice en éluant par 300 $cm^3$ d'un mélange toluène-diéthylamine-éthanol (60-20-20 en volumes) et en recueillant des fractions de 30 $cm^3$. Les fractions comprises entre 330 et 900 $cm^3$ sont concentrées à sec. Les cristaux jaunes obtenus sont dissous dans 25 $cm^3$ d'acétone et additionnés de 3 $cm^3$ d'une solution 3,4N d'acide chlorhydrique dans l'isopropanol. Le précipité formé est filtré sur verre fritté puis recristallisé dans 80 $cm^3$ d'un mélange éthanol-acétone (20-60 en volumes). Après addition de 10 $cm^3$ d'éther éthylique on obtient 2,05 g de chlorhydrate de N-{[(dihydro-3,4 2H-benzopyran-1 yl-4)-2 éthyl]-1 pipéridyl-4} fluoro-4 benzamide sous forme d'un solide blanc cristallisé fondant à 240 °C.

Le chlorhydrate de N-(pipéridyl-4) fluoro-4 benzamide peut être préparé de la manière suivante :

2,70 g de chlorhydrate de N-(benzyl-1 pipéridyl-4) fluoro-4 benzamide dans 50 $cm^3$ d'éthanol à 95 % sont hydrogénés à 60 °C, sous pression atmosphérique, en présence de 0,6 g d'hydroxyde de palladium. Après filtration sur Kieselguhr et concentration à sec sous pression réduite (5,2 kPa) on obtient des cristaux blancs qui sont utilisés tels quels dans l'étape suivante.

Le chlorohydrate de N-(benzyl-1 pipéridyl-4) fluoro-4 benzamide peut être préparé de la manière suivante :

2 g d'amino-4 benzyl pipéridine dissous dans 20 $cm^3$ de dichlorométhane sont agités pendants 2 heures à 0 °C avec 1,30 $cm^3$ du chlorure de l'acide fluoro-4 benzoïque en présence de 1,5 $cm^3$ de

triéthylamine. après 18 heures à température ambiante, le mélange réactionnel est lavé par une solution ammoniacale à 30 % puis à l'eau jusqu'à pH neutre. la phase organique est ensuite séchée sur sulfate de magnésium puis concentrée sous pression réduite (5,2 kPa).

Les cristaux jaunes obtenus sont purifiés par chromatographie sur colonne de 4 cm de diamètre contenant 70 g de gel de silice en éluant par 480 $cm^3$ d'un mélange toluène-diéthylamine-éthanol (80-10-10 en volumes) et en recueillant des fractions de 30 $cm^3$. Les fractions comprises entre 120 $cm^3$ et 4800 $cm^3$ sont concentrées à sec.

Les cristaux blancs obtenus sont repris par 20 $cm^3$ de dichlorométhane et 3 $cm^3$ d'une solution 3,5N d'acide chlorhydrique dans l'isopropanol. On obtient ainsi 2,70 g de chlorhydrate de N-(benzyl-1 pipéridyl-4) fluoro-4 benzamide fondant à 236 °C.

EXEMPLE 22

On opère comme à l'exemple 4, mais à partir de 0,70 g (bromo-2 éthyl)-4 dihydro-3,4 2H-benzopyranne, 0,75 g de chlorhydrate de N-phényl N-(pipéridyl-4) méthanesulfonamide, 0,75 g de carbonate de potassium sec et 0,35 g d'iodure de potassium dans 45 $cm^3$ de butanone-2. L'huile jaune obtenue est purifiée par chromatographie sur colonne de 4 cm de diamètre contenant 60 g de gel de silice en éluant par 660 $cm^3$ d'un mélange dichlorométhane-éthanol (95-5 en volumes) et en recueillant des fractions de 30 $cm^3$. Les fractions comprises entre 120 $cm^3$ et 660 $cm^3$ sont concentrées à sec. On reprend l'huile obtenue par 10 $cm^3$ d'éthanol et ajoute 0,35 g d'acide fumarique dissous dans 10 $cm^3$ d'éthanol. On concentre à sec, lave les cristaux à l'acétone et recristallise dans 20 $cm^3$ d'isopropanol.

On obtient ainsi 0,310 g de fumarate acide de {[(dihydro-3,4 2H-benzopyran-1 yl-4)-2 éthyl]-1 pipéridyl-4} N-phényl méthanesulfonamide sous forme d'un solide blanc fondant à 186 °C.

Le chlorhydrate de N-phényl N-(pipéridyl-4) méthanesulfonamide peut être préparé de la manière suivante :

1,16 g de chlorhydrate de N-(benzyl-1 pipéridyl-4) N-phényl méthanesulfonamide dans 50 $cm^3$ d'éthanol à 95 % sont hydrogénés à 60 °C, sous pression atmosphérique, en présence de 0,3 g d'hydroxyde de palladium.

Après filtration sur Kieselguhr et concentration à sec sous pression réduite (5,2 kPa) on obtient 0,758 g de cristaux blancs que l'on utilise tels quels dans l'étape suivante.

Le N-(benzyl-1 pipéridyl-4) N-phényl méthanesulfonamide peut être préparé de la manière suivante :

5,8 g de benzyl-1 anilino-4 pipéridine dissous dans 40 $cm^3$ de dichlorométhane sont agités sous argon pendant 7 heures à 0 °C avec 2 $cm^3$ de chlorure de l'acide méthanesulfonique et en présence de 3 $cm^3$ de triéthylamine. Après traitement comme décrit à l'exemple 21, on isole une huile qui est chromatographiée par CLHP (sur Waters Prep. 500 avec une colonne Prep. PAK de 5 cm de diamètre et 30 cm de longueur contenant de la silice 55-105 $\mu$) en utilisant comme éluant le mélange dichlorométhane-acétate d'isopropyle (5-1 en volumes) et en recueillant les fractions comprises entre 1500 $cm^3$ et 2350 $cm^3$.

On obtient ainsi 1,05 g de N-(benzyl-1 pipéridyl-4) N-phényl méthanesulfonamide sous forme d'un solide blanc fondant à 125 °C.

La benzyl-1 anilino-4 pipéridine a été préparée selon la méthode décrite dans la demande de brevet néerlandais 65 06 574.

EXEMPLE 23

On opère comme à l'exemple 4, mais à partir de 1,6 g de (bromo-2 éthyl)-4 dihydro-3,4 2H-benzopyranne, 2,34 g de chlorhydrate de N-phényl N-(pipéridyl-4) benzènesulfonamide, 1,8 g de carbonate de potassium sec et 0,8 g d'iodure de potassium dans 50 $cm^3$ de butanone-2.

On reprend l'huile obtenue dans le minimum d'éthanol et ajoute 1 g d'acide benzènesulfonique dissous dans l'éthanol. Le précipité blanc formé est filtré sur verre fritté puis recristallisé dans 80 $cm^3$ de mélange éthanol-méthanol (50-50 en volumes).

On obtient ainsi 2,45 g de benzènesulfonate de N-{[(dihydro-3,4 2H-benzopyran-1 yl-4)-2 éthyl]-1 pipéridyl-4} N-phényl benzènesulfonamide sous forme d'un solide blanc fondant à 150 °C.

Le chlorhydrate de N-phényl N-(pipéridyl-4) benzènesulfonamide peut être préparé comme décrit à l'exemple 22 pour le N-phényl N-(pipéridyl-4) méthanesulfonamide.

La présente invention concerne également les compositions pharmaceutiques constituées par un produit de formule générale (I) sous forme libre ou sous forme de sel d'addition avec un acide

pharmaceutiquement acceptable, à l'état pur ou sous forme d'une association avec tout autre produit pharmaceutiquement compatible, pouvant être inerte ou physiologiquement actif. Les compositions selon l'invention peuvent être utilisées par voie orale ou parentérale.

Comme compositions solides pour administration orale peuvent être utilisés des comprimés, des pilules, des poudres ou des granulés. Dans ces compositions, le produit actif selon l'invention (éventuellement associé à un autre produit pharmaceutiquement compatible) est mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser les émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pharmaceutiques selon l'invention qui réduisent les troubles du rythme cardiaque dus à des phénomènes de ré-entrée, traités ou non, sont particulièrement utiles en thérapeutique humaine dans les traitements consécutifs à l'infarctus du myocarde ainsi que dans les états angineux chroniques et les cardiopathies de type ischémique.

D'une façon générale, le médecin déterminera la posologie qu'il estime la plus appropriée en fonction de l'âge, du poids et des autres facteurs propres au sujet à traiter.

Généralement les doses sont comprises entre 0,25 et 1,5 g par jour, de produit actif par voie orale ou intraveineuse pour un adulte.

Les exemples suivants donnés à titre non limitatif, illustrent une composition selon l'invention.

<u>Exemple A</u>

On prépare des comprimés ayant la composition suivante :

| | |
|---|---|
| - oxalate acide de {[(dihydro-3,4 2H-benzopyran-1 yl-4))-2 éthyl]-1 pipéridyl-4} acétanilide..... | 161 mg |
| - lactose ..... | 50 mg |
| - excipient ..... | q.s.p. 250 mg |

<u>Exemple B</u>

On prépare les comprimés ayant la composition suivante :

| | |
|---|---|
| - chlorhydrate de benzoyl-4 [(dihydro-3,4 2H-benzopyran-1 yl-4)-2 éthyl]-1 pipéridine ..... | 165,6 mg |
| - lactose ..... | 50 mg |
| - excipient ..... | q.s.p. 250 mg |

**Revendications**

1 - Un nouveau dérivé du benzopyranne caractérisé en ce qu'il répond à la formule générale :

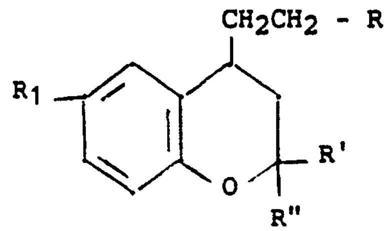

dans laquelle
- $R_1$ représente un atome d'hydrogène ou d'halogène ou un radical hydroxy, alcoyloxy, nitro, amino, alcoylsulfonamido ou acylamino,
- R représente
1) un radical de formule générale :

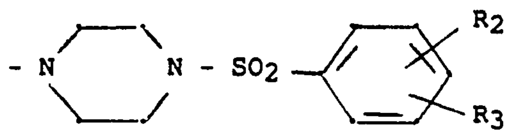

dans laquelle $R_2$ et $R_3$ identiques ou différents représentent des atomes d'hydrogène ou d'halogène ou des radicaux hydroxy, alcoyle, alcoyloxy, amino, alcoylsulfonamido ou nitro, ou bien
2) un radical de formule générale :

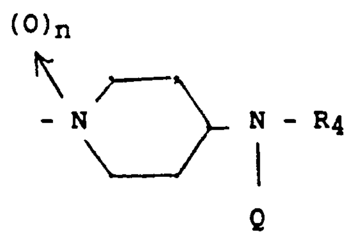

dans laquelle n égale 0 ou 1, $R_4$ est un atome d'hydrogène, un radical alcoyle ou un radical de structure :

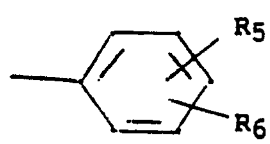

dans laquelle $R_5$ et $R_6$ sont des atomes d'hydrogène ou d'halogène ou un radical alcoyloxy et Q représente un radical acyle, alcoylsulfonyle ou un radical de structure :

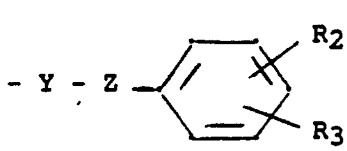

dans laquelle $R_2$ et $R_3$ sont définis comme ci-dessus, Y représente un radical carbonyle ou sulfonyle et Z représente une liaison simple ou un radical méthylène ou imino, ou bien
3) un radical de formule générale :

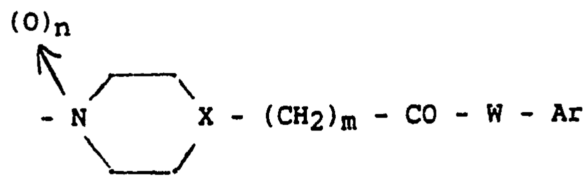

dans laquelle n égale 0 ou 1, m égale 0 à 2, X est un atome de carbone ou X peut être un atome d'azote si n = 0, W représente une liaison simple ou un radical imino et Ar représente un radical pyridyle, indolyle, quinolyle, alcoyl-2 quinolyle ou phényle éventuellement substitué par des radicaux $R_2$ et $R_3$ tels que définis ci-dessus, à condition que m soit autre que O lorsque X est un atome d'azote ou bien

4) un radical de formule générale :

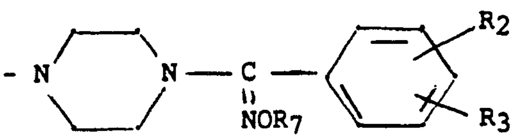 (VI)

dans laquelle $R_2$ et $R_3$ sont définis comme précédemment et $R_7$ représente un atome d'hydrogène ou un radical alcoyle, ou bien

5) un radical de formule :

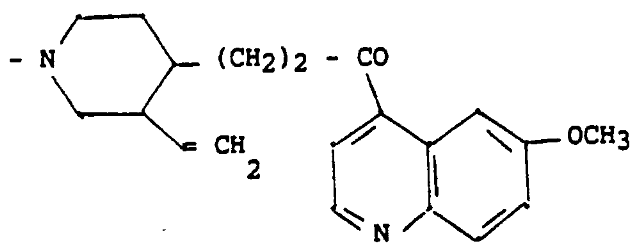

- R′ et R″ sont identiques et représentent des atomes d'hydrogène ou des radicaux alcoyle,

étant entendu que les radicaux et portions alcoyle et acyle cités ci-dessus contiennent 1 à 4 atomes de carbone en chaîne droite ou ramifiée, sous ses formes isomères et leurs mélanges, ainsi que ses sels d'addition avec les acides.

2- Un nouveau dérivé du benzopyranne selon la revendication 1, pour lequel $R_1$, R′ et R″ sont des atomes d'hydrogène, et R représente un radical tel que défini en 1) pour lequel $R_1$ et $R_3$ sont des atomes d'hydrogène, ou

R représente un radical tel que défini en 2) pour lequel n égal O, $R_4$ est un atome d'hydrogène ou un radical alcoyle ou un radical de structure (IVa) dans laquelle $R_5$ et $R_6$ sont des atomes d'hydrogène ou d'halogène, ou des radicaux alcoyloxy et Q représente un radical acétyle, méthylsulfonyle ou un radical de formule générale (IVb) dans laquelle Y est un radical carbonyle ou sulfonyle et Z est une liaison ou un radical méthylène ou imino, et $R_2$ et $R_3$ sont des atomes d'hydrogène ou d'halogène ou des radicaux alcoyle ou méthylsulfonamido, ou

R représente un radical tel que défini en 3) pour lequel n égal O, m égale 0 à 2, W est une liaison ou un radical imino, Ar est pyridyle, indolyle ou phényle éventuellement substitué par un atomes d'halogène, ou des radicaux alcoyle ou alcoyloxy et X est un atome de carbone ou d'azote, ou

R représente un radical tel que défini en 4) pour lequel $R_7$ est un atomes d'hydrogène.

3 - Le N-{[dihydro-3,4 2H-benzopyran-1 yl-4)-2 éthyl]-1 pipéridyl-4} acétanilide et ses sels ;

4 - La [(dihydro-3,4 2H-benzopyran-1 yl-4)-2 éthyl]-1 nicotinoyl-4 pipérazine et ses sels ;

5 - Le N-{[dihydro-3,4 2H-benzopyran-1 yl-4)-2 éthyl]-1 pipéridyl-4} N-phényl fluoro-4 benzamide et ses sels ;

6 - La benzoyl-4 [(dihydro-3,4 2H-benzopyran-1 yl-4)-2 éthyl]-1 pipéridine et ses sels ;

7 - Le {[dihydro-3,4 2H-benzopyran-1 yl-4)-2 éthyl]-1 pipéridyl-4} phényl méthanoxime et ses sels ;

8 - Procédé de préparation d'un nouveau dérivé du benzopyranne selon la revendication 1, caractérisé en ce que l'on fait agir un produit de formule générale :

H - R

ou son sel, dans laquelle R est défini comme dans la revendication 1 sous réserve que n soit égal à 0, et le cas échéant les radicaux amino et/ou hydroxy contenus dans R étant protégés, sur un dérivé du benzopyranne de formule générale :

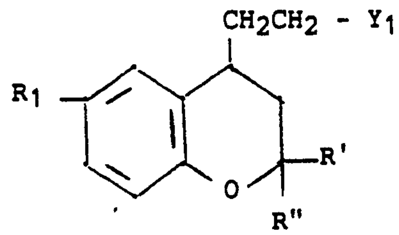

sous ses formes isomères ou leurs mélanges, dans laquelle $R_1$, $R'$ et $R''$ sont définis comme dans la revendication 1, le cas échéant $R_1$ étant protégé, et $Y_1$ représente un atome d'halogène ou un radical alcoylsulfonyloxy ou arylsulfonyloxy, éventuellement oxyde le produit obtenu pour préparer un produit selon la revendication 1 pour lequel n = 1 ou bien éventuellement transforme le produit obtenu en oxime, pour préparer un dérivé du benzopyranne selon la revendication 1 pour lequel R est défini en 4), lorsque l'on a obtenu la cétone correspondante pour laquelle R est un radical défini en 3) dans la revendication 1 (pour lequel m = O et -W-Ar est un radical phényl éventuellement substitué), puis élimine le cas échéant les radicaux protecteurs et transforme éventuel lement le produit obtenu en un sel d'addition avec un acide.

9 - Procédé de préparation selon la revendication 2, caractérisé en ce que l'on met en oeuvre un dérivé du benzopyranne de formule générale :

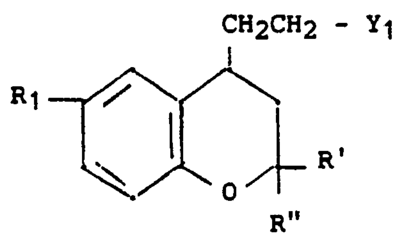

sous ses formes isomères ou leurs mélanges, dans laquelle $R_1$, $R'$ et $R''$ sont définis comme dans la revendication 1, et $Y_1$ est un atome de brome ou de chlore, ou un radical méthylsulfonyloxy ou p.toluènesulfonyloxy.

10 - Procédé de préparation d'un nouveau dérivé du benzopyranne selon la revendication 1 pour lequel les radicaux $R_1$, $R_2$ et/ou $R_3$ représentent un radical hydroxy, caractérisé en ce que l'on traite en milieu acide concentré le dérivé correspondant du benzopyranne selon la revendication 1 pour lequel le radical $R_1$, $R_2$ et/ou $R_3$ à transformer représente un radical alcoyloxy, puis transforme éventuellement le produit obtenu en un sel d'addition avec un acide.

11 - Procédé de préparation d'un nouveau dérivé du benzopyranne selon la revendication 1, pour lequel R est défini comme dans la revendication 1 en 1) ou 2) et les radicaux $R_1$, $R_2$ et/ou $R_3$ représentent des radicaux amino ou alcoylsulfonamido, ou pour lequel le radical $R_1$ représente un radical acylamino, caractérisé en ce que l'on effectue l'hydrogénation catalytique du dérivé correspondant du benzopyranne, selon la revendication 1, pour lequel le symbole $R_1$, $R_2$ et/ou $R_3$ à transformer représente un radical nitro, puis lorsque l'on veut obtenir un dérivé du benzopyranne pour lequel les radicaux $R_1$, $R_2$ et/ou $R_3$ sont définis comme ci-dessus à l'exception de représenter le radical amino, on transforme le dérivé aminé du benzopyranne obtenu, respectivement par sulfonylation ou par acylation, puis transforme éventuellement le produit obtenu en un sel d'addition avec un acide.

12 - Procédé de préparation d'un nouveau dérivé du benzopyranne selon la revendication 1, pour lequel R est défini comme dans la revendication 1 en 1) et 3), X étant un atome d'azote, caractérisé en ce que l'on fait agir un halogénure de formule générale :

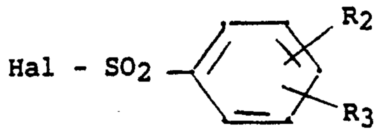

ou Hal - $(CH_2)_m$ - CO - W - Ar

dans laquelle $R_2$, $R_3$, W, Ar et m sont définis comme dans la revendication 1 et Hal représente un atome d'halogène, sur un dérivé du benzopyranne de formule générale :

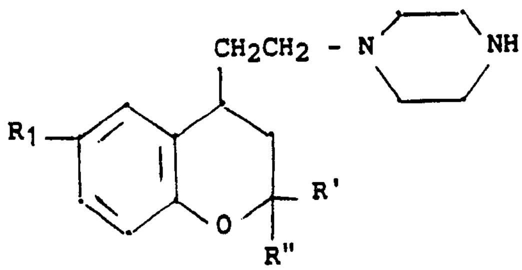

dans laquelle $R_1$, R′ et R″ sont définis comme dans la revendication 1, puis transforme éventuellement le produit obtenu en un sel d'addition avec un acide.

13 - Composition pharmaceutique caractérisée en ce qu'elle comprend un dérivé du benzopyranne selon la revendication 1, à l'état pur ou sous forme d'une association avec un ou plusieurs diluants ou adjuvants compatibles et pharmaceutiquement acceptables.

Revendication pour les Etats contractants suivants: ES, GR

Procédé de préparation d'un nouveau dérivé du benzopyranne de formule générale :

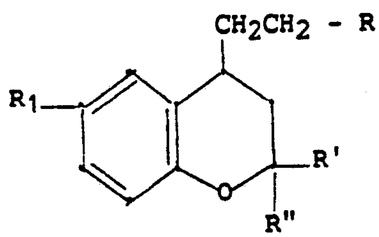

(I)

dans laquelle
- $R_1$ représente un atome d'hydrogène ou d'halogène ou un radical hydroxy, alcoyloxy, nitro, amino, alcoylsulfonamido ou acylamino,
- R représente
1) un radical de formule générale :

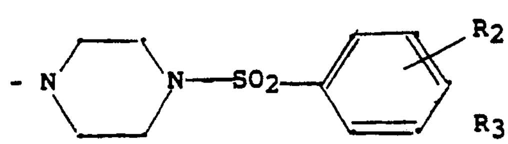

dans laquelle $R_2$ et $R_3$ identiques ou différents représentent des atomes d'hydrogène ou d'halogène ou des radicaux hydroxy, alcoyle, alcoyloxy, amino, alcoylsulfonamido ou nitro, ou bien
2) un radical de formule générale :

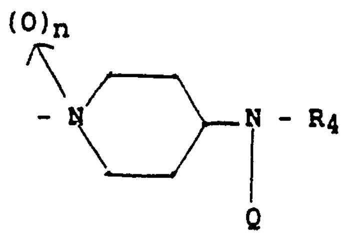

dans laquelle n égale 0 ou 1, $R_4$ est un atome d'hydrogène ou un radical alcoyle ou un radical de structure :

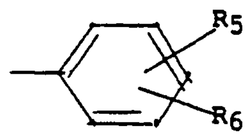

dans laquelle $R_5$ et $R_6$ sont des atomes d'hydrogène ou d'halogène ou un radical alcoyloxy et Q représente un radical acyle, alcoylsulfonyle ou un radical de structure :

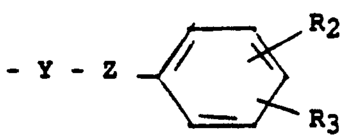

dans laquelle $R_2$ et $R_3$ sont définis comme ci-dessus, Y représente un radical carbonyle ou sulfonyle et Z représente une liaison simple ou un radical méthylène ou imino, ou bien

3) un radical de formule générale :

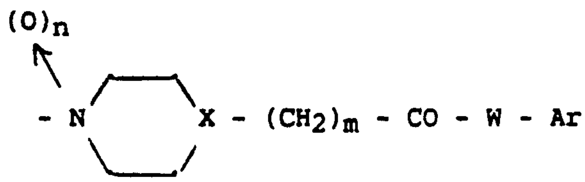

dans laquelle n égale 0 ou 1, m égale 0 à 2, X est un atome de carbone ou X peut être un atome d'azote si n = 0, W représente une liaison simple ou un radical imino et Ar représente un radical pyridyle, indolyle, quinolyle, alcoyl-2 quinolyle ou phényle éventuellement substitué par des radicaux $R_2$ et $R_3$ tels que définis ci-dessus, à condition que m soit autre que O lorsque X est un atome d'azote ou bien

4) un radical de formule générale :

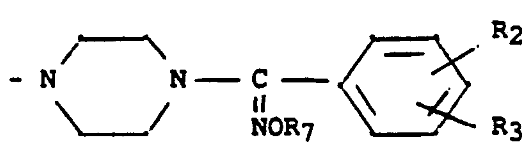

(VI)

dans laquelle $R_2$ et $R_3$ sont définis comme précédemment et $R_7$ est un atome d'hydrogène ou un radical alcoyle, ou bien

5) un radical de formule :

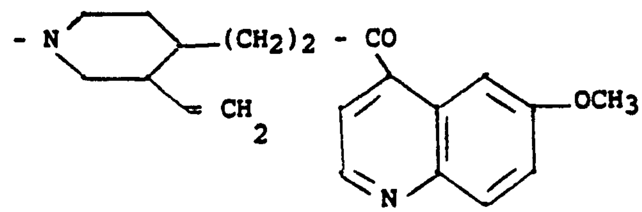

- R′ et R″ sont identiques et représentent des atomes d'hydrogène ou des radicaux alcoyle, étant entendu que les radicaux et portions alcoyle et acyle cités ci-dessus contiennent 1 à 4 atomes de carbone en chaîne droite ou ramifiée, sous ses formes isomères et leurs mélanges, ainsi que de ses sels d'addition avec les acides, caractérisé en ce que l'on fait agir un produit de formule générale :

H - R

ou son sel, dans laquelle R est défini comme dans la revendication 1 sous réserve que n soit égal à 0, et le cas échéant les radicaux amino et/ou hydroxy contenus dans R étant protégés, sur un dérivé du benzopyranne de formule générale :

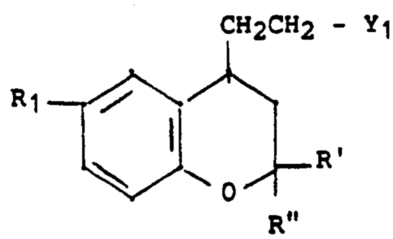

sous ses formes isomères ou leurs mélanges, dans laquelle $R_1$, R′ et R″ sont définis comme précédemment, le cas échéant $R_1$ étant protégé, et $Y_1$ représente un atome d'halogène ou un radical alcoylsulfonyloxy ou arylsulfonyloxy, éventuellement oxyde le produit obtenu pour préparer un produit pour lequel n = 1 ou bien éventuellement transforme le produit obtenu en oxime, pour préparer un dérivé du benzopyranne de formule (I) pour lequel R est défini en 4), lorsque l'on a obtenu la cétone correspondante pour laquelle R est un radical défini en 3) dans la revendication 1 (pour lequel m = O et -W-Ar est un radical phényle éventuellement substitué), puis élimine le cas échéant les radicaux protecteurs, ou bien

lorsque les radicaux $R_1$, $R_2$ et/ou $R_3$ représentent un radical hydroxy, on traite en milieu acide concentré le dérivé correspondant du benzopyranne de formule (I) pour lequel le radical $R_1$, $R_2$ et/ou $R_3$ à transformer représente un radical alcoyloxy, ou bien lorsque R est défini comme précédemment en 1) ou 2) et les radicaux $R_1$, $R_2$ et/ou $R_3$ représentent des radicaux amino ou alcoylsulfonamido, ou lorsque le radical $R_1$ représente un radical acylamino, on effectue l'hydrogénation catalytique du dérivé correspondant du benzopyranne de formule (I) pour lequel le symbole $R_1$, $R_2$ et/ou $R_3$ à transformer représente un radical nitro, puis lorsque l'on veut obtenir un dérivé du benzopyranne pour lequel les radicaux $R_1$, $R_2$ et/ou $R_3$ sont définis comme ci-dessus à l'exception de représenter le radical amino, on transforme le dérivé aminé du benzopyranne obtenu, respectivement par sulfonylation ou par acylation, ou bien

lorsque R est défini comme précédemment en 1) et 3), X étant un atome d'azote, on fait agir un halogénure de formule générale :

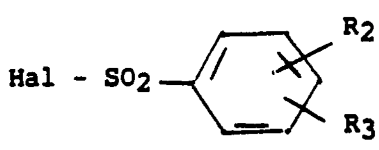 ou $Hal - (CH_2)_m - CO - W - Ar$

dans laquelle $R_2$, $R_3$, W, Ar et m sont définis comme précédemment et Hal représente un atome d'halogène, sur un dérivé du benzopyranne de formule générale :

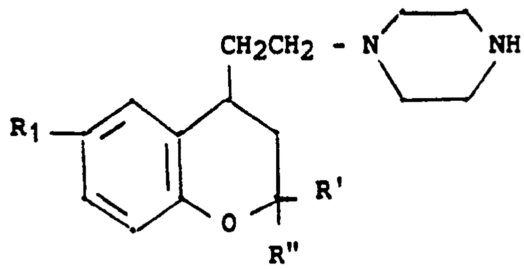

dans laquelle $R_1$, $R'$ et $R''$ sont définis comme précédemment, puis transforme éventuellement le produit obtenu en un sel d'addition avec un acide.

Office européen des brevets

# RAPPORT DE RECHERCHE EUROPEENNE

Numero de la demande

EP 90 40 0153

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,A | EP-A-0 114 374 (BAYER)<br>* Pages 1-8,58-62; revendications *<br>----- | 1,2,8, 13 | C 07 D 405/06<br>C 07 D 405/12<br>C 07 D 311/58<br>A 61 K 31/395 |
| | | | **DOMAINES TECHNIQUES RECHERCHES (Int. Cl.5)** |
| | | | C 07 D 405/00<br>C 07 D 311/00 |

Le présent rapport a été établi pour toutes les revendications

| Lieu de la recherche | Date d'achèvement de la recherche | Examinateur |
|---|---|---|
| LA HAYE | 26-02-1990 | FRANCOIS J.C.L. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)